# EUROPEAN PATENT SPECIFICATION

(11) **EP 2 453 895 B1**
(45) Date of publication and mention of the grant of the patent: **23.05.2018**
(21) Application number: 10736929.0
(22) Date of filing: 16.07.2010
(51) Int. Cl.: A61K 31/472, A61K 31/485, A61K 45/06, A61P 25/00, A61P 29/00, C07D 217/20, C07D 221/28, C07D 489/00, C07D 489/02, C07D 489/08, C07D 489/10

(54) **(+) - MORPHINANS AS ANTAGONISTS OF TOLL-LIKE RECEPTOR 9 AND THERAPEUTIC USES THEREOF**
(+) - MORPHINANE ALS ANTAGONISTEN DES TOLL-LIKE-REZEPTORS 9 UND IHRE THERAPEUTISCHE VERWENDUNG
(+) MORPHINANES EN TANT QU'ANTAGONISTES DU RÉCEPTEUR DE TYPE TOLL 9 ET UTILISATIONS THÉRAPEUTIQUES DE CELLES-CI

(30) Priority: 16.07.2009 US 226015 P; 16.12.2009 US 286877 P
(43) Date of publication of application: 23.05.2012
(73) Proprietor: Mallinckrodt LLC, Hazelwood, MO 63042 (US)
(72) Inventor: TRAWICK, Bobby, N., Florissant Missouri 63034 (US); BERBERICH, David, W., St. Peters Missouri 63376 (US)
(74) Representative: Jones, Nicholas Andrew
(86) International application number: PCT/US2010/042210
(87) International publication number: WO 2011/009015

(56) References cited:
- WO-A1-2007/127624
- WO-A1-2009/012005
- WO-A1-2010/096790
- WO-A1-2012/008984
- WO-A2-2009/023819
- WO-A2-2009/059048
- WO-A2-2010/039209
- CN-A- 101 066 948
- CN-A- 101 265 266
- US-A- 4 521 601
- US-A- 5 668 285
- US-A1- 2009 156 817
- US-A1- 2009 156 818
- IIJIMA I ET AL: "Studies in the (+)-Morphinan Series. 4. A Markedly Improved Synthesis of (+)-Morphine" JOURNAL OF ORGANIC CHEMISTRY, AMERICAN CHEMICAL SOCIETY, EASTON.; US LNKD- DOI:10.1021/JO00401A038, vol. 43, no. 7, 1 January 1978 (1978-01-01), pages 1462-1463, XP001156547 ISSN: 0022-3263
- HELLEWELL S B ET AL: "A sigma-like binding site in rat pheochromocytoma (PC12) cells: decreased affinity for (+)-benzomorphans and lower molecular weight suggest a different sigma receptor form from that of guinea pig brain" BRAIN RESEARCH, ELSEVIER, AMSTERDAM, NL LNKD- DOI:10.1016/0006-8993(90)91143-5, vol. 527, no. 2, 17 September 1990 (1990-09-17), pages 244-253, XP024268162 ISSN: 0006-8993 [retrieved on 1990-09-17]
- YE XIANRONG ET AL: "Synthesis and anti-inflammatory analgesic activities of sinomenine derivatives" YAO HSUEH HSUEH PAO - ACTA PHARMACEUTICA SINICA, YAOXUE XUEBAO, CN, vol. 39, no. 3, 1 January 2004 (2004-01-01), pages 180-183, XP001525528 ISSN: 0513-4870
- IIJIMA I ET AL: "Studies in the (+) morphinan series. V. Synthesis and biological properties of (+) naloxone" JOURNAL OF MEDICINAL CHEMISTRY, AMERICAN CHEMICAL SOCIETY, WASHINGTON, US LNKD- DOI:10.1021/JM00202A018, vol. 21, no. 4, 1 January 1978 (1978-01-01), pages 398-400, XP002428747 ISSN: 0022-2623
- KITAMURA M ET AL: "GENERAL ASYMMETRIC SYNTHESIS OF ISOQUINOLINE ALKALOIDS. ENANTIOSELECTIVE HYDROGENATION OF ENAMIDES CATALYZED BY BINAP- RUTHENIUM(II) COMPLEXES" JOURNAL OF ORGANIC CHEMISTRY, AMERICAN CHEMICAL SOCIETY, EASTON.; US LNKD- DOI:10.1021/JO00081A007, vol. 59, no. 2, 1 January 1994 (1994-01-01), pages 297-310, XP002050844 ISSN: 0022-3263
- LIU C P ET AL: "Inhibition of (S)-armepavine from Nelumbo nucifera on autoimmune disease of MRL/MpJ-lpr/lpr mice" EUROPEAN JOURNAL OF PHARMACOLOGY, ELSEVIER BV, NL LNKD- DOI:10.1016/J.EJPHAR.2005.11.062, vol. 531, no. 1-3, 15 February 2006 (2006-02-15), pages 270-279, XP025169511 ISSN: 0014-2999 [retrieved on 2006-02-15]
- FIALIP J ET AL: "Study of the antinociceptive effect of tetrahydro-papaveroline derivatives: Interactions with opioids" LIFE SCIENCES, vol. 59, no. 11, 1996, pages 133-139, XP002606195 ISSN: 0024-3205
- HUTCHINSON MARK R ET AL: "Non-stereoselective reversal of neuropathic pain by naloxone and naltrexone: involvement of toll-like receptor 4 (TLR4)" EUROPEAN JOURNAL OF NEUROSCIENCE, vol. 28, no. 1, July 2008 (2008-07), pages 20-29, XP002606196 ISSN: 0953-816X
- MAHMOOD S ET AL: "56 Aacetaminophen Hepatotoxicity Is Dependent On TLR9 and Can Be Reduced By a TLR9 Antagonist", GASTROENTEROLOGY, ELSEVIER, PHILADELPHIA, PA, vol. 134, no. 4, 1 April 2008 (2008-04-01) , pages A-752, XP023435311, ISSN: 0016-5085, DOI: 10.1016/S0016-5085(08)63514-3 [retrieved on 2008-04-01]

## Description

### FIELD OF THE INVENTION

The present disclosure generally relates to compounds and methods for treating inflammation, pain, and other disorders. In particular, the disclosure relates to (+)-morphinan compounds comprising Toll-like receptor 9 (TLR9) antagonist activity and methods of using the compounds to treat conditions associated with pain and inflammation.

### BACKGROUND OF THE INVENTION

Activated glial cells contribute to the development and maintenance of several disease states. Of particular interest is the negative impact of activated glial cells in the areas of chronic and acute pain, inflammatory disorders, autoimmune disorders, neurodegenerative disorders, and cancer. Glial cells have been shown to express numerous Toll-like receptors (TLRs), which are a family of highly conserved transmembrane proteins of high functional importance in the innate immune system. TLRs are activated by pathogen-associated molecular patterns (PAMPs) such as lipopolysaccharide (LPS) from bacterial cell walls, unmethylated CpG-containing DNA of viruses, and a wide variety of additional microbial components. Activation of TLRs in the central nervous system is known to initiate protective pro-inflammatory signaling cascades as part of the first line of defense against invading pathogens. Additionally, it has been reported that chronic administration of morphine or other opioid-receptor agonists activates glial cells, causing the release of pro-inflammatory factors that counter the pain-relieving effects of the opioid. Activated glial cells have also been shown to play a role in driving chronic pain states such as neuropathic pain. Given these newly identified roles for glial cells in pain, there is a need for the development of clinically useful agents that target glial cell activation as a means of pain control. WO2009/059048 describes 4,5-epoxymorphinans and derivatives thereof. Mahmood et al., Gastroenterology, Elsevier, vol. 134, no. 4, 1 April 2008, pages A-752 is directed to the use of TLR9 antagonists in reducing acetaminophen-induced hepatotoxicity. WO 2007/127624 is directed to methods and compositions for reducing the invasiveness of cancer cells that express a member of the TLR9 subfamily. WO2009/023819 relates to TLR9 antagonist compounds and their therapeutic or prophylactic use.

### SUMMARY OF THE INVENTION

The present invention provides (+)-morphinan compounds that inhibit the activation of Toll-like receptor 9 (TLR9), and consequently block glial cell activation. The compounds of the invention, therefore, may be used to treat conditions such as traumatic pain, neuropathic pain, inflammatory disorders, acetaminophen toxicity, autoimmune disorders, neurodegenerative disorders, and cancer.

One aspect of the invention encompasses a compound or a pharmaceutically acceptable salt thereof selected from the group consisting of I-2, I-3, I-4, I-6, I-7, I-8, I-12, I-13, I-14, I-21, I-22, I-26, I-27, I-29, II-1, II-2, II-4, II-5, II-8, II-9, II-10, II-11, II-15, II-16, II-19, II-20, II-21, II-22, II-23, II-25, II-26, II-28, II-29, II-30, II-31, II-33, II-35, II-36, II-37, II-41, II-42, II-43, II-44, II-46, II-48, II-51, II-53, II-55, II-57, II-59, II-60, II-70, II-72, II-75, II-79, II-83, II-89, II-90, II-91, II-92, II-93, II-94, II-97, II-99, II-100, III-2, III-3, III-4, and III-5.

Another aspect of the invention provides a method for inhibiting TLR9 activation. The method comprises contacting a in-vitro cell expressing TLR9 with a compound or a pharmaceutically acceptable salt thereof selected from the group consisting of I-1, I-2, I-3, I-4, I-6, I-7, I-8, I-12, I-13, I-14, I-21, I-22, I-25, I-26, I-27, I-28, I-29, II-1, II-2, II-4, II-5, II-7, II-10, II-11, II-15, II-19, II-21, II-23, II-25, II-26, II-29, II-35, II-36, II-37, II-38, II-42, II-43, II-60, II-62, II-63, II-71, II-72, II-74, II-76, II-78, II-82, II-86, II-88, II-90, II-91, II-92, II-93, II-95, II-96, II-98, II-99, III-1, III-2, III-3, III-4, and III-5.

Other aspects and features of the invention are detailed below.

### DESCRIPTION OF THE FIGURES

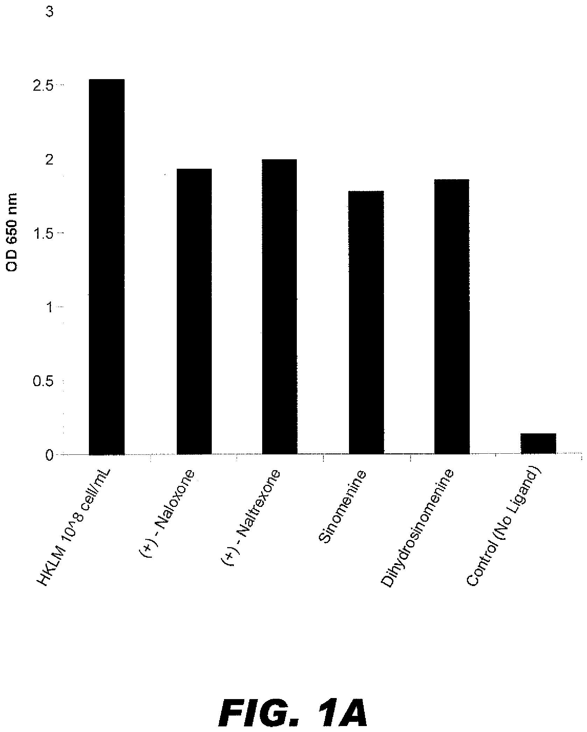

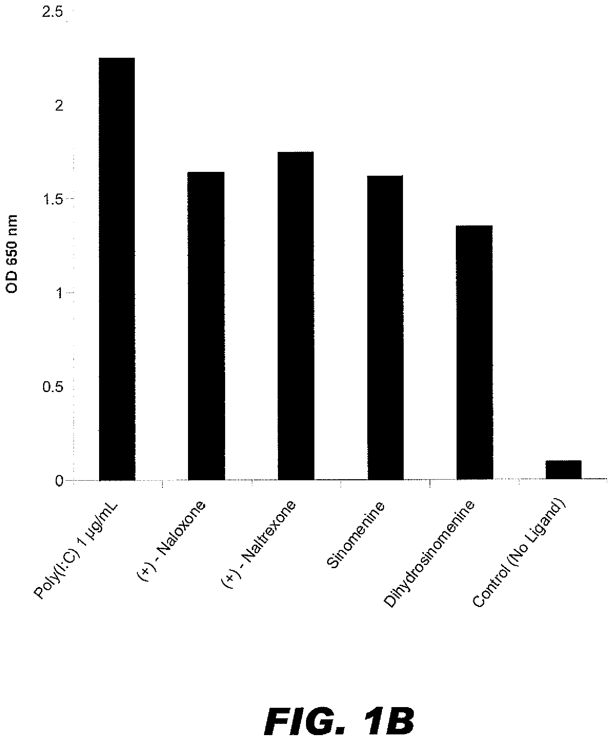

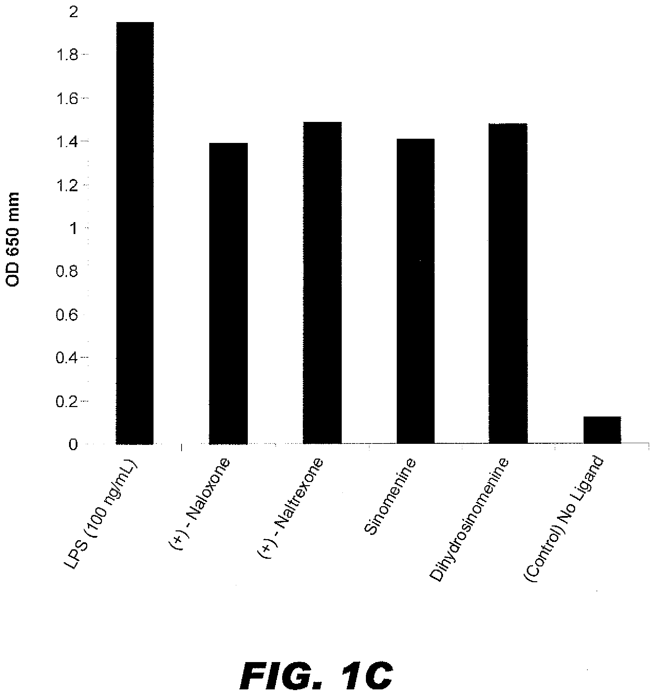

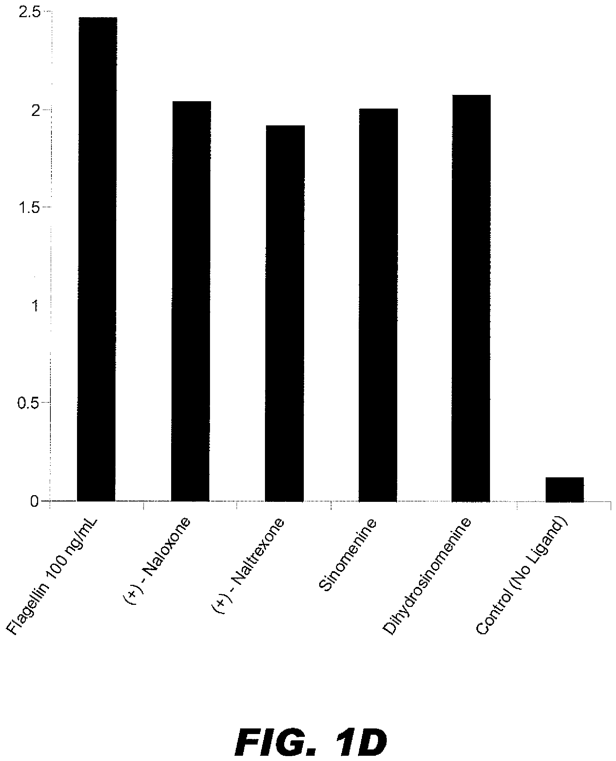

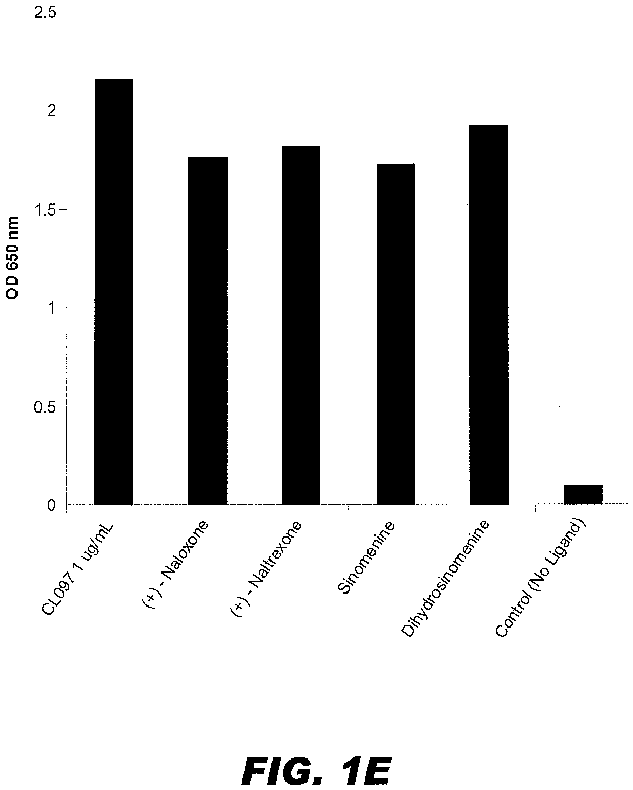

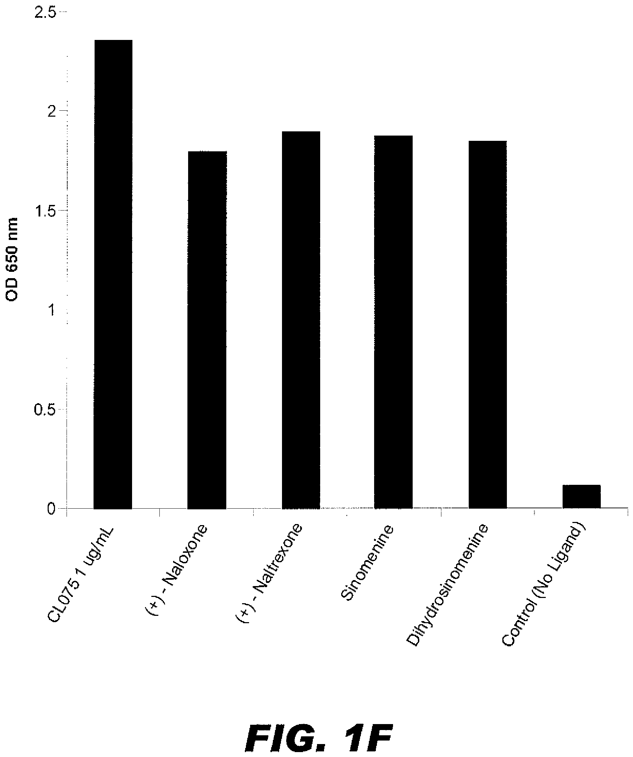

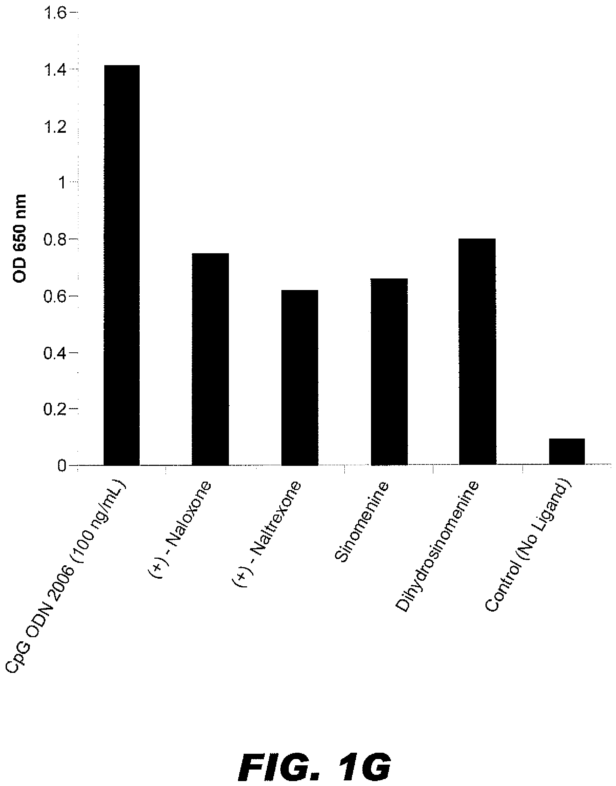

**FIG. 1** presents Toll-like receptor (TLR) antagonist screenings. Each panel presents the results for a particular TLR. Plotted is the activity of a secreted alkaline phosphatase reporter system in optical density (OD) at 650 nm for each treatment condition, which was tested in duplicate. All agents were tested at 10 µM. **(A)** presents TLR2 antagonist screening in which TLR2 was stimulated with HKLM at 10⁸ cell/ml. (**B)** presents TLR3 antagonist screening in which TLR3 was stimulated with poly(I:C) at 1 µg/ml. **(C)** presents TLR4 antagonist screening in which TLR4 was stimulated with LPS at 100 ng/ml. (**D)** presents TLR5 antagonist screening in which TLR5 was stimulated with Flagellin at 100 ng/ml. **(E)** presents TLR7 antagonist screening in which TLR7 was stimulated with CL097 at 1 µg/ml. **(F)** presents TLR8 antagonist screening in which TLR8 was stimulated with CL075 at 1 µg/ml. **(G)** presents TLR9 antagonist screening in which TLR9 was stimulated with CpG ODN 2006 at 100 ng/ml.

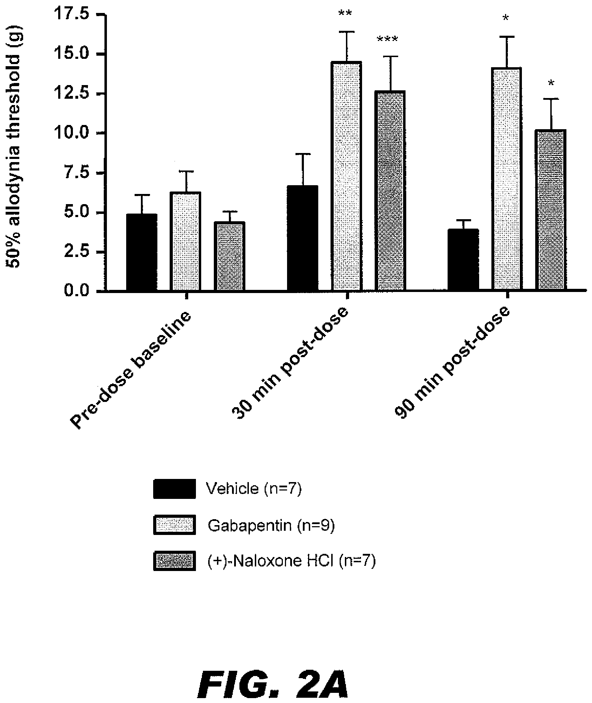

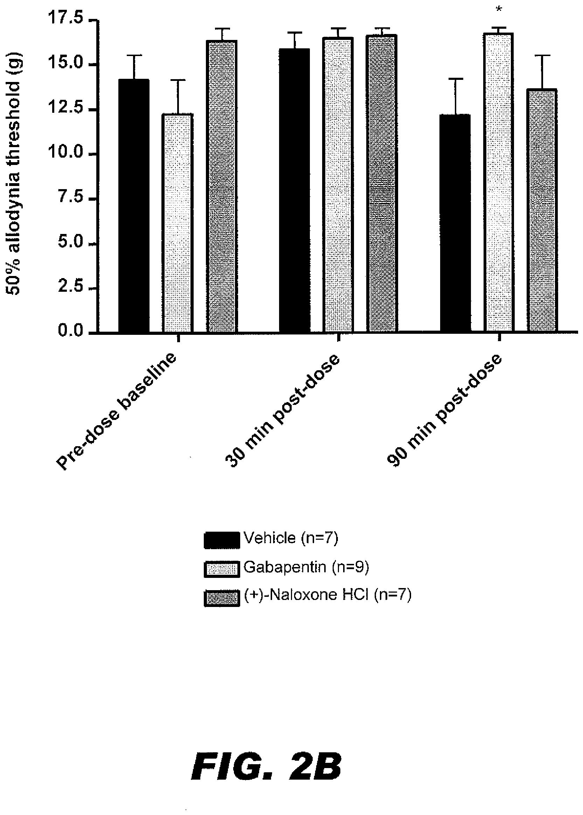

**FIG. 2** illustrates the analgesic effects of (+)-naloxone on mechanical allodynia in rats. Plotted is 50% allodynia threshold at three timepoints for each treatment group on Day 14. Bars represent mean +/- SEM. **(A)** presents data for the left (affected) paw. **(B)** presents data for the right (unaffected) paw. *p<0.05, **p<0.01, ***p<0.001 vs. vehicle.

### DETAILED DESCRIPTION OF THE INVENTION

It has been discovered that certain (+)-morphinans block the activation of TLR9 and, consequently, the activation of glial cells. Thus, (+)-morphinans comprising TLR9 antagonist activity may be used to treat pain, as well as other conditions associated with pain and inflammation. It has also been discovered that the inhibition of TLR9 activation may be used as a screening tool to identify (+)-morphinans that may be therapeutically effective in treating conditions such as traumatic pain, neuropathic pain, inflammatory disorders, acetaminophen toxicity, autoimmune disorders, neurodegenerative disorders, and cancer. Accordingly, the present invention provides (+)-morphinans comprising TLR9 antagonist activity and methods for inhibiting the activation of TLR9.

### (I) (+)-Morphinans Comprising TLR9 Antagonist Activity

### (a) compounds comprising Formula (I)

One aspect of the present invention is the provision of (+)-morphinans comprising TLR9 antagonist activity. The (+)-morphinan is a compound represented by formulae (I), (II) or (III).

Formula (I) is a compound having one of the following structures:

**Table A. Compounds of Formula (I)**

| Name | Structure | Name | Structure |
|---|---|---|---|
| | | I-2 | 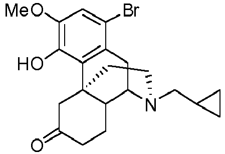 |
| I-3 | 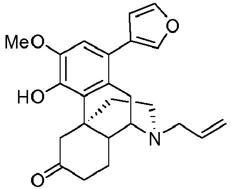 | I-4 | 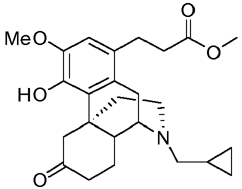 |
| | | I-6 | 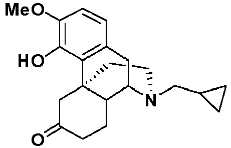 |
| I-7 | 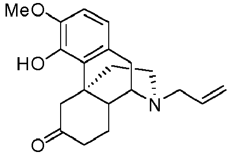 | I-8 | 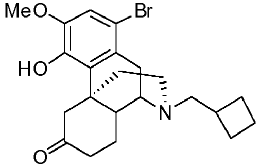 |
| | | I-12 | 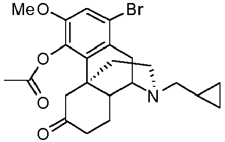 |
| I-13 | 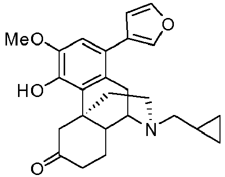 | I-14 | 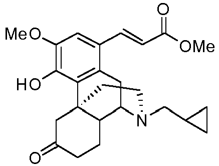 |
| I-21 | 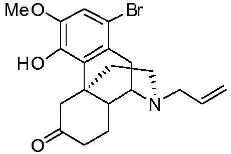 | I-22 | 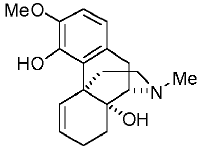 |
| | | I-26 | 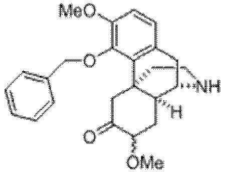 |
| I-27 | 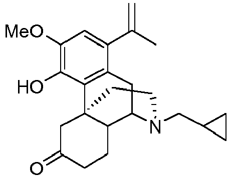 | | |
| I-29 | 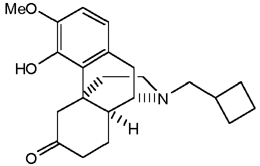 | | |

### (b) compounds of Formula (II)

The (+)-morphinan compounds of Formula (II) or a pharmaceutically acceptable salt thereof have the following structures:

**Table B. Compounds of Formula (II)**

| Name | Structure | Name | Structure |
|---|---|---|---|
| II-1 | 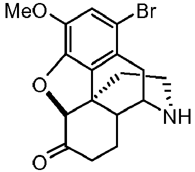 | II-2 | 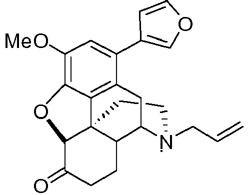 |
| | | II-4 | 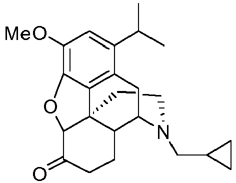 |
| II-5 | 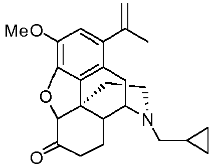 | | |
| | | II-8 | 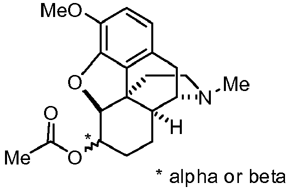 |
| II-9 | 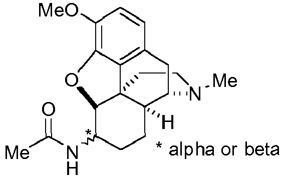 | II-10 | 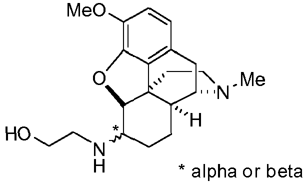 |
| II-11 | 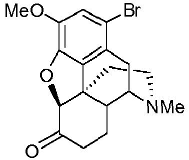 | | |
| II-15 | 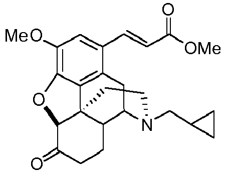 | II-16 | 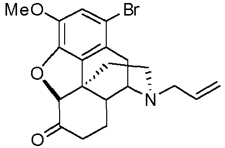 |
| II-19 | 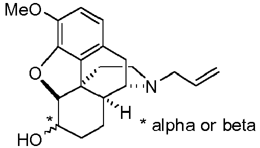 | II-20 | 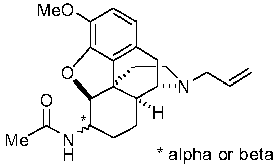 |
| II-21 | 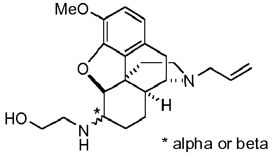 | II-22 | 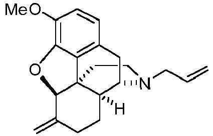 |
| II-23 | 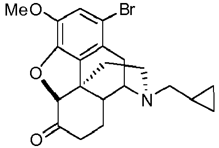 | | |
| II-25 | 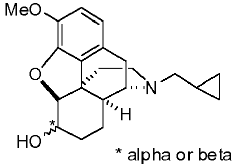 | II-26 | 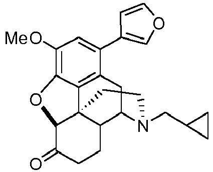 |
| | | II-28 | 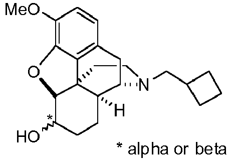 |
| II-29 | 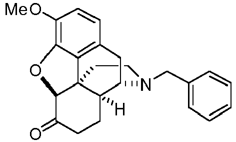 | II-30 | 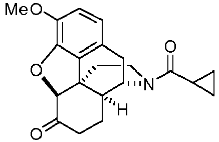 |
| II-31 | 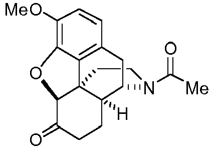 | | |
| II-33 | 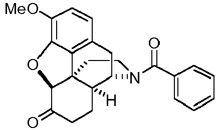 | | |
| II-35 | 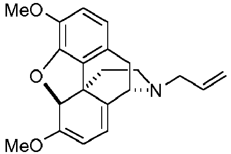 | II-36 | 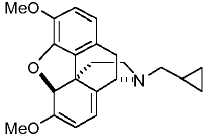 |
| II-37 | 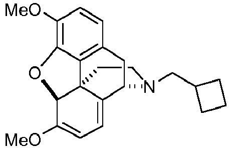 | | |
| II-41 | 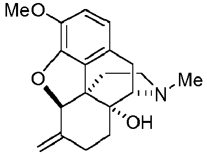 | II-42 | 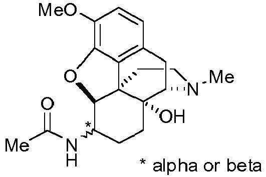 |
| II-43 | 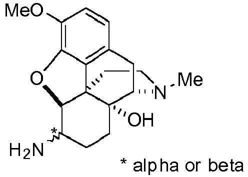 | II-44 | 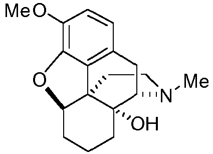 |
| | | II-46 | 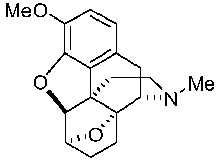 |
| | | II-48 | 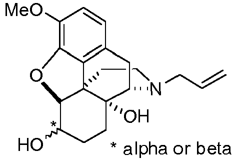 |
| II-51 | 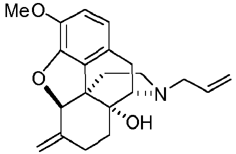 | | |
| II-53 | 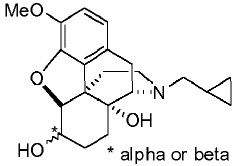 | | |
| II-55 | 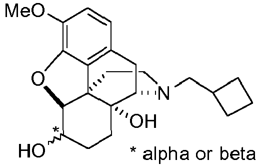 | | |
| II-57 | 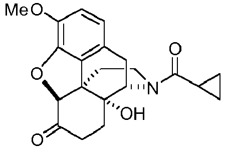 | | |
| II-59 | 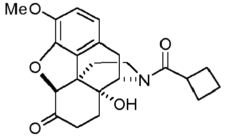 | II-60 | 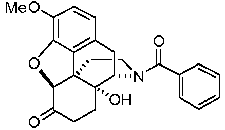 |
| | | II-70 | 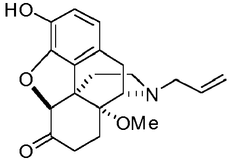 |
| | | II-72 | 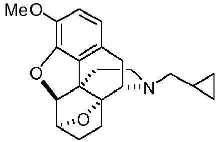 |
| II-75 | 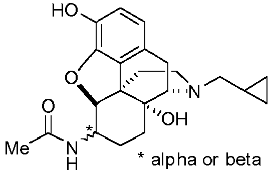 | | |
| II-79 | 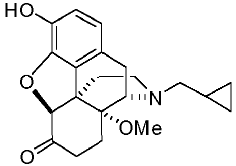 | | |
| II-83 | 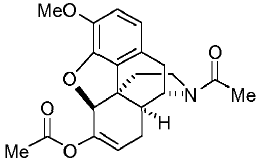 | | |
| | | II-90 | 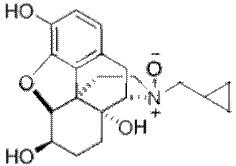 |
| II-91 | 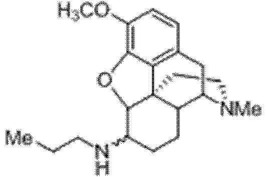 | II-92 | 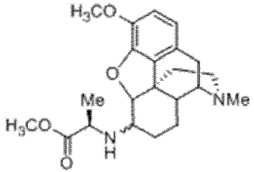 |
| II-93 | 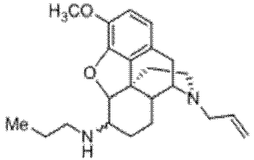 | II-94 | 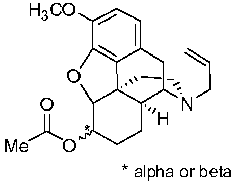 |
| II-97 | 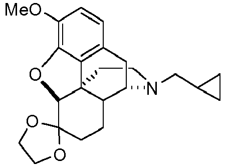 | | |
| II-99 | 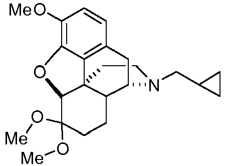 | II-100 | 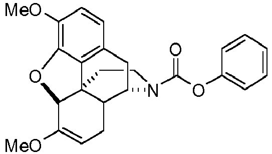 |

### (c) compounds of Formula (III)

The compound having TLR9 antagonist activity represented by Formula (III) or a pharmaceutically acceptable salt thereof is selected from the following formulae:

**Table C. Compounds of Formula (III)**

| Name | Structure | Name | Structure |
|---|---|---|---|
| | | III-2 | 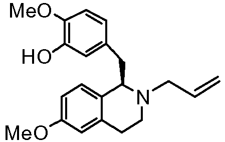 |
| III-3 | 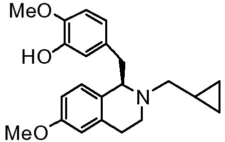 | III-4 | 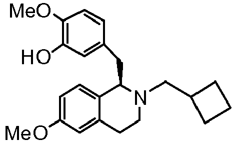 |
| III-5 | 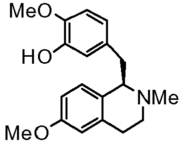 | | |

### (d) pharmaceutically acceptable salts

The compounds of Formulas (I), (II), or (III) may be provided as pharmaceutically-acceptable salts. The term "pharmaceutically-acceptable salt" refers to a salt commonly used to form an alkali metal salt or addition salt of a free acid or a free base. The nature of the salt may vary, provided that it is pharmaceutically acceptable. Suitable pharmaceutically acceptable acid addition salts of compounds of the present invention may be prepared from an inorganic acid or from an organic acid. Examples of such inorganic acids are hydrochloric, hydrobromic, hydroiodic, nitric, carbonic, sulfuric and phosphoric acid. Appropriate organic acids may be selected from aliphatic, cycloaliphatic, aromatic, araliphatic, heterocyclic, carboxylic and sulfonic classes of organic acids, examples of which are formic, acetic, propionic, succinic, glycolic, gluconic, lactic, malic, tartaric, citric, ascorbic, glucuronic, maleic, fumaric, pyruvic, aspartic, glutamic, benzoic, anthranilic, mesylic, 4-hydroxybenzoic, phenylacetic, mandelic, embonic (pamoic), methanesulfonic, ethanesulfonic, benzenesulfonic, pantothenic, 2-hydroxyethanesulfonic, toluenesulfonic, sulfanilic, cyclohexylaminosulfonic, stearic, algenic, hydroxybutyric, salicylic, galactaric and galacturonic acid. Suitable pharmaceutically-acceptable base addition salts of compounds of the present invention include metallic salts made from aluminum, calcium, lithium, magnesium, potassium, sodium and zinc or organic salts made from N, N'-dibenzylethylenediamine, chloroprocaine, choline, diethanolamine, ethylenediamine, meglumine- (N-methylglucamine) and procaine. All of these salts may be prepared by conventional means from the corresponding compound by reacting, for example, the appropriate acid or base with the any of the compounds of the invention.

### (e) stereochemistry

Each of the compounds of Formulas (I), (II), or (III) has a (+) orientation with respect to the rotation of polarized light. More specifically, each chiral carbon has an R or an S configuration. As will be appreciated by a skilled artisan, the R or S configuration for a given compound will change depending on the particular Formula, i.e., (I), (II), or (III), and the compound's substitution pattern.

### (II) Therapeutic Uses of TLR9 Antagonists

Also described is a method for treating a condition in a subject. In general, the method comprises administering to the subject at least one (+)-morphinan comprising TLR9 antagonist activity either alone or in combination with at least one additional therapeutic agent. In general, the compound is selected from Formulas (I), (II), (III), or a pharmaceutically acceptable salt thereof. A variety of disorders or disease states may be treated with the compounds of the invention. Suitable disorders and diseases that may be treated include pain conditions, inflammatory disorders, acetaminophen toxicity, autoimmune disorders, neurodegenerative disorders, and cancer.

The subject to be treated may be any subject having one of the indicated conditions. Alternatively, the subject to be treated may be in need of treatment for the condition. That is, the subject has been diagnosed with the condition or is at risk for developing the condition. The subject may be diagnosed with the condition using diagnostic or clinical tests that are well known. Furthermore, those of skill in the art appreciate that different diagnostic or clinical tests are used to diagnosis the different diseases or disorders. The diagnostic tools include, without limit, physical examination, patient history, screening tests, laboratory tests, molecular tests, genomic tests, imaging tools, physical tests and mental tests. Since the perception of pain may be quite subjective, tools such as the McGill Pain Questionnaire may be used to assess the quality of pain (e.g., sharp, stabbing, squeezing, etc.), and the intensity of pain may be quantified using a numerical scale that ranges from 0 to 10. Skilled diagnosticians are familiar with other indicators of pain.

In general, the subject will be a human. However, other mammalian subjects may be used. Suitable mammalian subjects include; companion animals, such as cats and dogs; livestock animals, such as cows, pigs, horses, sheep, and goats; zoo animals; and research animals, such as non-human primates and rodents.

### (a) conditions

### (i) pain conditions

The compound comprising Formulas (I), (II),(III), or a pharmaceutically acceptable salt thereof may be used alone or in combination with at least one additional therapeutic agent for the treatment of pain. As used herein, the term "pain" refers to the unpleasant sensory and emotional experience associated with actual or perceived tissue damage by a noxious stimulus. The pain may be acute or chronic pain. For example, the pain may be traumatic or inflammatory pain, which results from injury to non-neural tissue. Non-limiting examples of traumatic or inflammatory pain include arachnoiditis, arthritis, back pain, burn pain, central pain syndrome, cancer pain, headaches (including migraines, cluster, and tension headaches); head and facial pain, muscle pain (including fibromyalgia), myofascial pain syndromes; reflex sympathetic dystrophy syndrome, repetitive stress injuries, sciatica, shingles and other skin disorders, sports injuries, spinal stenosis, surgical pain, temporomandibular disorders, trauma, and/or vascular disease or injury.

Alternatively, the pain may be neuropathic pain, which results from injury to or inflammation of the central or peripheral nervous system. Neuropathic pain may occur in any part of the body and is frequently described as a hot, burning sensation, which can be devastating to the affected individual. Neuropathic pain may be acute or chronic; it may result from diseases that affect nerves (such as diabetes), from trauma, surgical procedures, arthritis, AIDS, burn injuries, cerebral or lumbar spine disease, fibromyalgia, post-eschemic pain, tumors, viral neuralgias, or, because chemotherapy drugs can affect nerves, it may be a consequence of cancer treatment. Among the many neuropathic pain conditions are diabetic neuropathy (which results from nerve damage secondary to vascular problems that occur with diabetes); reflex sympathetic dystrophy syndrome, which may follow injury; phantom limb and post-amputation pain, which may result from the surgical removal of a limb; post-herpetic neuralgia, which may occur after an outbreak of shingles; and complex regional pain syndrome or central pain syndrome, which may result from trauma to the brain or spinal cord.

Characteristic symptoms of neuropathic pain include hyperesthesia (i.e., enhanced sensitivity to a natural stimulus); allodynia (i.e., widespread tenderness or hypersensitivity to tactile stimuli); hyperalgesia (i.e., abnormal sensitivity to pain); spontaneous burning pain; and/or phantom pain (i.e., perception of pain that is non-existent). Hyperesthesia involves an unusual increased or altered sensitivity to sensory stimuli, including for example, acoustic, cerebral, gustatory, muscular, olfactory, onelric, optic, or tactile. As an example, a painful sensation from a normally painless touch stimulus. Allodynia involves an intensified, unpleasant, and painful perception of stimuli triggered by heat or by contact, which is based on a lowering of the pain threshold for these stimuli, including, for example, a non-noxious stimulus to normal skin. Hyperalgesia involves the excessive perception of a variety of stimuli, again based on a lowering of the pain threshold and thus an abnormally increased pain sense, including for example, auditory or muscular stimuli. Phantom pain involves a perception of pain in a limb that is non-existent, such as perceived pain in a limb that has been amputated, i.e. phantom limb syndrome.

### (ii) inflammatory disorders

The compound of Formulas (I), (II), (III), or a pharmaceutically acceptable salt thereof may be used alone or in combination with at least one additional therapeutic agent for the treatment of inflammation in a subject. For example, the inflammatory disorder may be arthritis including, but not limited to, rheumatoid arthritis, spondyloarthropathies, gouty arthritis, osteoarthritis, systemic lupus erythematosus, or juvenile arthritis. In some embodiments, the inflammation may be associated with asthma, allergic rhinitis, sinus diseases, bronchitis, tuberculosis, acute pancreatitis, sepsis, infectious diseases, menstrual cramps, premature labor, tendinitis, bursitis, skin-related conditions such as psoriasis, eczema, atopic dermatitis, urticaria, dermatitis, contact dermatitis, and burns, or from post-operative inflammation including from ophthalmic surgery such as cataract surgery and refractive surgery. The inflammatory disorder may be a gastrointestinal condition such as inflammatory bowel disease, Crohn's disease, gastritis, irritable bowel syndrome, chronic cholecystitis, or ulcerative colitis. The inflammation may be associated with diseases such as vascular diseases, migraine headaches, periarteritis nodosa, thyroiditis, aplastic anemia, Hodgkin's disease, sclerodoma, rheumatic fever, type I diabetes, neuromuscular junction disease including myasthenia gravis, white matter disease including multiple sclerosis, sarcoidosis, nephrotic syndrome, Behcet's syndrome, polymyositis, gingivitis, nephritis, hypersensitivity, swelling occurring after injury, myocardial ischemia, allergic rhinitis, respiratory distress syndrome, systemic inflammatory response syndrome (SIRS), cancer-associated inflammation, reduction of tumor-associated angiogenesis, endotoxin shock syndrome and atherosclerosis. The inflammatory disorder may be associated with an ophthalmic disease, such as retinitis, retinopathies, uveitis, ocular photophobia, or of acute injury to the eye tissue. The inflammation may be a pulmonary inflammation, such as that associated with viral infections or cystic fibrosis, chronic obstructive pulmonary disease, or acute respiratory distress syndrome. The inflammatory disorder may also be associated with tissue rejection, graft v. host diseases, delayed-type hypersensitivity, as well as immune-mediated and inflammatory elements of CNS diseases such as Alzheimer's, Parkinson's and multiple sclerosis.

### (iii) acetaminophen toxicity

The compound of Formulas (I), (II), (III), or a pharmaceutically acceptable salt thereof may also be alone or in combination with at least one other therapeutic agent to treat acetaminophen toxicity (also known as paracetamol toxicity). High levels of acetaminophen may lead to damage of the liver (i.e., acetaminophen-induced hepatotoxicity) or the kidney (i.e., acetaminophen-induced nephrotoxicity). Acetaminophen toxicity may result from an acute overdose of acetaminophen or a chronic overdose acetaminophen. The amount of ingested acetaminophen at which toxicity occurs may be reduced upon chronic ethanol use, malnourishment, or diminished nutritional status, fasting, or viral illness with dehydration, or use of certain pharmaceutical agents that interact with the enzyme systems that metabolize acetaminophen.

Acetaminophen-induced hepatotoxicity may be manifested by cellular oxidative damage, mitochondrial dysfunction, and a subsequent inflammatory response. Cellular damage may be monitored by elevated levels of serum alanine transaminase (ALT) or serum aspartate transaminase (AST), and the inflammatory response may be monitored by increased levels of pro-interleukin(IL)-1beta transcript levels. Acetaminophen-induced hepatotoxicity also may lead to hepatocellular injury, death, and centrilobular (zone III) liver necrosis. Similar enzymatic reactions occur in the kidney, and may contribute to some degree of extra-hepatic organ dysfunction.

### (iv) autoimmune disorders

The compound of Formulas (I), (II), (III), or a pharmaceutically acceptable salt thereof may also be alone or in combination with at least one other therapeutic agent to treat an autoimmune disease or disorder. The autoimmune disorder may be systemic, such as Lupus, wherein many tissues or organs are affected or damaged. Alternatively, the autoimmune disorder may be localized, such as type I diabetes mellitus, wherein a single organ or tissue is damaged or affected. Non-limiting examples of autoimmune disorders include acute disseminated encephalomyelitis (ADEM), Addison's disease, alopecia areata, antiphospholipid antibody syndrome (APS), autoimmune hemolytic anemia, autoimmune hepatitis, autoimmune inner ear disease, bullous pemphigoid, celiac disease, Chagas disease, chronic obstructive pulmonary disease, Crohn's disease, dermatomyositis, diabetes mellitus type 1, endometriosis, Goodpasture's syndrome, Graves' disease, Guillain-Barre syndrome (GBS), Hashimoto's thyroiditis, hidradenitis suppurativa, Kawasaki disease, IgA nephropathy, idiopathic thrombocytopenic purpura, interstitial cystitis, lupus erythematosus (Lupus), mixed connective tissue disease, morphea, multiple sclerosis, myasthenia gravis, narcolepsy, neuromyotonia, pemphigus vulgaris, pernicious anemia, psoriasis, psoriatic arthritis, polymyalgia rheumatica, polymyositis, primary biliary cirrhosis, rheumatoid arthritis and juvenile rheumatoid arthritis, schizophrenia, schleroderma, sclerosing cholangitis, Sjogren's syndrome, stiff person syndrome, temporal arteritis/giant cell arteritis, ulcerative colitis, vasculitis, vitiligo, and Wegener's granulomatosis.

### (v) neurodegenerative disorders

The compound of Formulas (I), (II), (III), or a pharmaceutically acceptable salt thereof may be used alone or in combination with at least one other therapeutic agent to treat a neurodegenerative disorder. Non-limiting examples of neurodegenerative disorders include adrenal leukodystrophy, aging-related disorders and dementias, alcoholism, Alexander's disease, Alper's disease, Alzheimer's disease, amyotrophic lateral sclerosis (Lou Gehrig's Disease), ataxia telangiectasia, Batten disease (also known as Spielmeyer-Vogt-Sjögren-Batten disease), bovine spongiform encephalopathy (BSE), canavan disease, cerebral palsy, Cockayne syndrome, corticobasal degeneration (CBD), Creutzfeldt-Jakob disease, familial fatal insomnia, frontotemporal lobar degeneration, frontal temporal dementias (FTDs), Huntington's disease, HIV-associated dementia, Kennedy's disease, Krabbe's disease, Lewy body disease, neuroborreliosis, Machado-Joseph disease (spinocerebellar ataxia type 3), multiple system atrophy, multiple sclerosis, narcolepsy, Niemann Pick disease, Parkinson disease, Pelizaeus-Merzbacher disease, Pick's disease, primary lateral sclerosis, progressive supranuclear palsy (PSP), psychotic disorders, Refsum's disease, Sandhoff disease, Schilder's disease, schizoaffective disorder, schizophrenia, stroke, subacute combined degeneration of spinal cord secondary to pernicious anemia, spinocerebellar ataxia, spinal muscular atrophy, Steele-Richardson-Olszewski disease, Tabes dorsalis, and toxic encephalopathy.

### (vi) cancers

The compound of Formulas (I), (II), (III), or a pharmaceutically acceptable salt thereof may be used in combination with a chemotherapeutic agent to treat a neoplasm or a cancer. The neoplasm may be malignant or benign, the cancer may be primary or metastatic; the neoplasm or cancer may be early stage or late stage. Non-limiting examples of neoplasms or cancers that may be treated include acute lymphoblastic leukemia, acute myeloid leukemia, adrenocortical carcinoma, AIDS-related cancers, AIDS-related lymphoma, anal cancer, appendix cancer, astrocytomas (childhood cerebellar or cerebral), basal cell carcinoma, bile duct cancer, bladder cancer, bone cancer, brainstem glioma, brain tumors (cerebellar astrocytoma, cerebral astrocytoma/malignant glioma, ependymoma, medulloblastoma, supratentorial primitive neuroectodermal tumors, visual pathway and hypothalamic gliomas), breast cancer, bronchial adenomas/carcinoids, Burkitt lymphoma, carcinoid tumors (childhood, gastrointestinal), carcinoma of unknown primary, central nervous system lymphoma (primary), cerebellar astrocytoma, cerebral astrocytoma/malignant glioma, cervical cancer, childhood cancers, chronic lymphocytic leukemia, chronic myelogenous leukemia, chronic myeloproliferative disorders, colon cancer, cutaneous T-cell lymphoma, desmoplastic small round cell tumor, endometrial cancer, ependymoma, esophageal cancer, Ewing's sarcoma in the Ewing family of tumors, extracranial germ cell tumor (childhood), extragonadal germ cell tumor, extrahepatic bile duct cancer, eye cancers (intraocular melanoma, retinoblastoma), gallbladder cancer, gastric (stomach) cancer, gastrointestinal carcinoid tumor, gastrointestinal stromal tumor, germ cell tumors (childhood extracranial, extragonadal, ovarian), gestational trophoblastic tumor, gliomas (adult, childhood brain stem, childhood cerebral astrocytoma, childhood visual pathway and hypothalamic), gastric carcinoid, hairy cell leukemia, head and neck cancer, hepatocellular (liver) cancer, Hodgkin lymphoma, hypopharyngeal cancer, hypothalamic and visual pathway glioma (childhood), intraocular melanoma, islet cell carcinoma, Kaposi sarcoma, kidney cancer (renal cell cancer), laryngeal cancer, leukemias (acute lymphoblastic, acute myeloid, chronic lymphocytic, chronic myelogenous, hairy cell), lip and oral cavity cancer, liver cancer (primary), lung cancers (non-small cell, small cell), lymphomas (AIDS-related, Burkitt, cutaneous T-cell, Hodgkin, non-Hodgkin, primary central nervous system), macroglobulinemia (Waldenström), malignant fibrous histiocytoma of bone/osteosarcoma, medulloblastoma (childhood), melanoma, intraocular melanoma, Merkel cell carcinoma, mesotheliomas (adult malignant, childhood), metastatic squamous neck cancer with occult primary, mouth cancer, multiple endocrine neoplasia syndrome (childhood), multiple myeloma/plasma cell neoplasm, mycosis fungoides, myelodysplastic syndromes, myelodysplastic/myeloproliferative diseases, myelogenous leukemia (chronic), myeloid leukemias (adult acute, childhood acute), multiple myeloma, myeloproliferative disorders (chronic), nasal cavity and paranasal sinus cancer, nasopharyngeal carcinoma, neuroblastoma, non-Hodgkin lymphoma, non-small cell lung cancer, oral cancer, oropharyngeal cancer, osteosarcoma/malignant fibrous histiocytoma of bone, ovarian cancer, ovarian epithelial cancer (surface epithelial-stromal tumor), ovarian germ cell tumor, ovarian low malignant potential tumor, pancreatic cancer, pancreatic cancer (islet cell), paranasal sinus and nasal cavity cancer, parathyroid cancer, penile cancer, pharyngeal cancer, pheochromocytoma, pineal astrocytoma, pineal germinoma, pineoblastoma and supratentorial primitive neuroectodermal tumors (childhood), pituitary adenoma, plasma cell neoplasia, pleuropulmonary blastoma, primary central nervous system lymphoma, prostate cancer, rectal cancer, renal cell carcinoma (kidney cancer), renal pelvis and ureter transitional cell cancer, retinoblastoma, rhabdomyosarcoma (childhood), salivary gland cancer, sarcoma (Ewing family of tumors, Kaposi, soft tissue, uterine), Sézary syndrome, skin cancers (nonmelanoma, melanoma), skin carcinoma (Merkel cell), small cell lung cancer, small intestine cancer, soft tissue sarcoma, squamous cell carcinoma, squamous neck cancer with occult primary (metastatic), stomach cancer, supratentorial primitive neuroectodermal tumor (childhood), T-Cell lymphoma (cutaneous), testicular cancer, throat cancer, thymoma (childhood), thymoma and thymic carcinoma, thyroid cancer, thyroid cancer (childhood), transitional cell cancer of the renal pelvis and ureter, trophoblastic tumor (gestational), unknown primary site (adult, childhood), ureter and renal pelvis transitional cell cancer, urethral cancer, uterine cancer (endometrial), uterine sarcoma, vaginal cancer, visual pathway and hypothalamic glioma (childhood), vulvar cancer, Waldenström macroglobulinemia, and Wilms tumor (childhood).

### (b) treatment formulations

### (i) formulations comprising at least one (+)-morphinan TLR9 antagonist

The treatment method may comprise administering to the subject at least one (+)-morphinan comprising TLR9 antagonist activity. In general, the TLR9 antagonist is a compound of Formulas (I), (II), (III), or a pharmaceutically acceptable salt thereof. Suitable pharmaceutically acceptable salts are detailed above in section (I)(d).

The compounds of the present invention may be formulated into pharmaceutical compositions and administered by a number of different means that will deliver a therapeutically effective dose. Such compositions may be administered orally, parenterally, by inhalation spray, rectally, intradermally, intrathecally, transdermally, or topically in dosage unit formulations containing conventional nontoxic pharmaceutically acceptable carriers, adjuvants, and vehicles as desired. Topical administration may also involve the use of transdermal administration such as transdermal patches or iontophoresis devices. Formulation of therapeutic agents is discussed in, for example, Gennaro, A. R., Remington's Pharmaceutical Sciences, Mack Publishing Co., Easton, Pa. (18th ed, 1995), and Liberman, H. A. and Lachman, L., Eds., Pharmaceutical Dosage Forms, Marcel Dekker Inc., New York, N.Y. (1980).

Preparations for oral administration generally contain inert excipients in addition to the active pharmaceutical ingredient. Oral preparations may be enclosed in gelatin capsules or compressed into tablets. Common excipients used in such preparations include pharmaceutically compatible fillers/diluents such as microcrystalline cellulose, hydroxypropyl methylcellulose, starch, lactose, sucrose, glucose, mannitol, sorbitol, dibasic calcium phosphate, or calcium carbonate; binding agents such as alginic acid, carboxymethylcellulose, microcrystalline cellulose, gelatin, gum tragacanth, or polyvinylpyrrolidone; disintegrating agents such as alginic acid, cellulose, starch, or polyvinylpyrrolidone; lubricants such as calcium stearate, magnesium stearate, talc, silica, or sodium stearyl fumarate; glidants such as colloidal silicon dioxide; sweetening agents such as sucrose or saccharin; flavoring agents such as peppermint, methyl salicylate, or citrus flavoring; coloring agents; and preservatives such as antioxidants (e.g., vitamin A, vitamin C, vitamin E, or retinyl palmitate), citric acid, or sodium citrate. Oral preparations may also be administered as aqueous suspensions, elixirs, or syrups. For these, the active ingredient may be combined with various sweetening or flavoring agents, coloring agents, and, if so desired, emulsifying and/or suspending agents, as well as diluents such as water, ethanol, glycerin, and combinations thereof.

For parenteral administration (including subcutaneous, intradermal, intravenous, intramuscular, and intraperitoneal), the preparation may be an aqueous or an oil-based solution. Aqueous solutions may include a sterile diluent such as water, saline solution, a pharmaceutically acceptable polyol such as glycerol, propylene glycol, or other synthetic solvents; an antibacterial and/or antifungal agent such as benzyl alcohol, methyl paraben, chlorobutanol, phenol and thimerosal; an antioxidant such as ascorbic acid or sodium bisulfite; a chelating agent such as etheylenediaminetetraacetic acid; a buffer such as acetate, citrate, or phosphate; and/or an agent for the adjustment of tonicity such as sodium chloride, dextrose, or a polyalcohol such as mannitol or sorbitol. The pH of the aqueous solution may be adjusted with acids or bases such as hydrochloric acid or sodium hydroxide. Oil-based solutions or suspensions may further comprise sesame, peanut, olive oil, or mineral oil.

For topical (e.g., transdermal or transmucosal) administration, penetrants appropriate to the barrier to be permeated are generally included in the preparation. Transmucosal administration may be accomplished through the use of nasal sprays, aerosol sprays, tablets, or suppositories, and transdermal administration may be via ointments, salves, gels, patches, or creams as generally known in the art.

The amount of agent that is administered to the subject can and will vary depending upon the type of agent, the subject, and the particular mode of administration. Those skilled in the art will appreciate that dosages may also be determined with guidance from Goodman & Goldman's The Pharmacological Basis of Therapeutics, Tenth Edition (2001), Appendix II, pp. 475-493, and the Physicians' Desk Reference.

### (ii) combination formulations

The treatment method may comprise administering to the subject a combination formulation comprising at least one (+)-morphinan TLR9 antagonist and at least one other therapeutic agent. In general, the TLR9 antagonist is a compound of Formulas (I), (II), (III), or a pharmaceutically acceptable salt thereof. It is envisioned that when the combination formulation comprises more than one additional therapeutic agent, the therapeutic agents may be drawn from one of the classes listed below, or the therapeutic agents may be drawn from different classes listed below.

The compound of Formulas (I), (II), (III), or a pharmaceutically acceptable salt thereof may be administered in combination with an analgesic agent. The analgesic may be an (-)-opioid analgesic. Alternatively, the analgesic may be a non-opioid analgesic. Non-limiting examples of suitable opioid analgesics include buprenorphine, butorphanol, codeine, dihydrocodeine, dihydromorphine, etorphine, fentanyl, hydrocodone, hydromorphone, levophanol, meperidine, methadone, morphine, nalbuphine, norcodeine, normorphine, oxycodone, oxymorphone, pentazocine, and propoxyphene. In some combinations comprising an opioid analgesic, the concentration or dose of the opioid analgesic in the combination formulation may be sub-analgesic. Examples of suitable non-opioid analgesics include without limit acetylsalicylic acid, acetaminophen (paracetamol), ibuprofen, ketoprofen, indomethacin, diflunisol, naproxen, ketorolac, dichlophenac, tolmetin, sulindac, phenacetin, piroxicam, and mefamanic acid. The analgesic may comprise a combination of an opiate analgesic and a non-opioid analgesic. For example, acetaminophen may be combined with codeine, hydrocodone, oxycodone, propoxyphene, or another opioid analgesics. The combination may comprise a compound of Formulas (I), (II), or (III), and acetaminophen. The concentration of acetaminophen in such a combination may be lower than in currently available acetaminophen combination formulations.

The compound of Formulas (I), (II), (III), or a pharmaceutically acceptable salt thereof may be administered in combination with an anti-inflammatory agent. The anti-inflammatory agent may be a glucocorticoid steroid such as the naturally occurring hydrocortisone (cortisol), or synthetic glucocorticoids such as prednisone, prednisolone, methylprednisolone, dexamethasone, betamethasone, triamcinolone, beclometasone, fludrocortisones, deoxycorticosterone, alclometasone, fluocinonide, aldosterone, and derivatives thereof. Alternatively, the anti-inflammatory agent may be a non-steroidal anti-inflammatory agent (NSAID). Non-limiting examples of suitable NSAIDs include acetylsalicylic acid (aspirin), celecoxib, choline magnesium salicylate, Cox-2 inhibitors, diclofenac, diflunisal, etodolac, fenoprofen, flufenisal, flurbiprofen, ibuprofen, indomethacin, ketoprofen, ketorolac, meclofenamate, mefenamate, nabumetone, naproxen, oxaprozin, phenylbutazone, piroxicam, salsalate, sulindac, tolmetin, valdecoxib, and zomepirac.

The compound of Formulas (I), (II), (III), or a pharmaceutically acceptable salt thereof may be administered in combination with an antibiotic agent. Non-limiting examples of suitable antibiotic agents include aminoglycosides such as, e.g., amikacin, gentamicin, kanamycin, neomycin, netilmicin, streptomycin, and tobramycin; a carbecephem such as loracarbef; carbapenems such as, e.g., certapenem, imipenem, and meropenem; cephalosporins such as, e.g., cefadroxil cefazolin, cephalexin, cefaclor, cefamandole, cephalexin, cefoxitin, cefprozil, cefuroxime, cefixime, cefdinir, cefditoren, cefoperazone, cefotaxime, cefpodoxime, ceftazidime, ceftibuten, ceftizoxime, and ceftriaxone; macrolides such as, e.g., azithromycin, clarithromycin, dirithromycin, erythromycin, and troleandomycin; monobactam; penicillins such as, e.g., amoxicillin, ampicillin, carbenicillin, cloxacillin, dicloxacillin, nafcillin, oxacillin, penicillin G, penicillin V, piperacillin, and ticarcillin; polypeptides such as, e.g., bacitracin, colistin, and polymyxin B; quinolones such as, e.g., ciprofloxacin, enoxacin, gatifloxacin, levofloxacin, lomefloxacin, moxifloxacin, norfloxacin, ofloxacin, and trovafloxacin; sulfonamides such as, e.g., mafenide, sulfacetamide, sulfamethizole, sulfasalazine, sulfisoxazole, and trimethoprim-sulfamethoxazole; tetracyclines such as, e.g., demeclocycline, doxycycline, minocycline, and oxytetracycline); and an antimicrobial agent such as, e.g., ketoconazole, amoxicillin, cephalexin, miconazole, econazole, acyclovir, and nelfinavir.

The compound of Formulas (I), (II), (III), or a pharmaceutically acceptable salt thereof may be administered in combination with an agent used to treat acetaminophen toxicity. Suitable agents include acetylcysteine (also called N-acetylcysteine), glutathione, and activated charcoal.

The compound of Formulas (I), (II), (III), or a pharmaceutically acceptable salt thereof may be administered in combination with an autoimmune therapeutic agent. Non-limiting examples of suitable autoimmune therapeutic agents include immunosuppressants such as azathioprine, chlorambucil, cyclophosphamide, cyclosporine, mycophenolate, or methotrexate; corticosteroids such as prednisone; the psoriasis treatment agent alefacept; TNF blockers such as etanercept, infliximab, or adalimumab; white blood cell blockers such as abatacept or ritaximab; the leprosy drug clofazimine; and chemotherapeutic agents such as vorinostat.

The compound of Formulas (I), (II), (III), or a pharmaceutically acceptable salt thereof may be administered in combination with a neurodegenerative disorder therapeutic agent. Typically, the neurodegenerative disorder therapeutic agent is tailored to the specific neurodegenerative disorder to be treated. Suitable therapeutic agents for the treatment of Parkinson disease include, without limit, levadopa (i.e., L-DOPA); a decarboxylase inhibitor such as carbidopa; a direct acting dopamine agonist such bromocriptine, pergolide, ropinirole or pramipexole; a dopamine uptake inhibitor such as amantadine; an anticholinergic such as trihexyphenidyl or benztropine mesylate; a monoamine oxidase B inhibitor such as L-deprenyl; a catechol-O-methyltranferase inhibitor such as tolcapone; spheramine; and combinations thereof. Non-limiting examples of suitable therapeutic agents for the treatment of Alzheimer's disease include cholinesterase inhibitors such as donepezil, rivastigmine and galantamine; NMDA receptor antagonists such as memantine; and Alzheimer's specific agents such as tramiprosate, tarenflubil and phenserine. Targeted therapeutic agents used to treat Huntington's disease include, without limit, tetrabenazine and xenazine. Non-limiting examples of therapeutic agents targeted to treat amyotrophic lateral sclerosis (ALS) include riluzole and mecasermin rinfabate.

The compound of Formulas (I), (II), (III), or a pharmaceutically acceptable salt thereof may be administered in combination with a chemotherapeutic agent. The chemotherapeutic agent may be a cytotoxic agent that affects rapidly dividing cells in general, or it may be a targeted therapeutic agent that affects the deregulated proteins of cancer cells. For example, the chemotherapeutic agent may be an alkylating agent, an anti-metabolite, an anti-tumor antibiotic, an anti-cytoskeletal agent, a topoisomerase inhibitor, an anti-hormonal agent, a targeted therapeutic agent, or a combination thereof. Non-limiting examples of alkylating agents include altretamine, benzodopa, busulfan, carboplatin, carboquone, carmustine, chlorambucil, chlornaphazine, cholophosphamide, chlorozotocin, cisplatin, cyclosphosphamide, dacarbazine (DTIC), estramustine, fotemustine, ifosfamide, improsulfan, lomustine, mechlorethamine, mechlorethamine oxide hydrochloride, melphalan, meturedopa, nimustine, novembichin, phenesterine, piposulfan, prednimustine, ranimustine; temozolomide, thiotepa, triethylenemelamine, trietylenephosphoramide, triethylenethiophosphaoramide, trimethylolomelamine, trofosfamide, uracil mustard and uredopa. Suitable anti-metabolites include, but are not limited to aminopterin, ancitabine, azacitidine, 6-azauridine, capecitabine, carmofur, cytarabine or cytosine arabinoside (Ara-C), dideoxyuridine, denopterin, doxifluridine, enocitabine, floxuridine, fludarabine, 5-fluorouracil (5-FU), gemcetabine, leucovorin (folinic acid), 6-mercaptopurine, methotrexate, pemetrexed, pteropterin, thiamiprine, trimetrexate, and thioguanine. Non-limiting examples of suitable anti-tumor antibiotics include aclacinomysin, actinomycin, adriamycin, authramycin, azaserine, bleomycins, cactinomycin, calicheamicin, carabicin, caminomycin, carzinophilin, chromomycins, dactinomycin, daunorubicin, detorubicin, 6-diazo-5-oxo-L-norleucine, doxorubicin, epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, and zorubicin. Non-limiting examples of suitable anti-cytoskeletal agents include colchicines, docetaxel, macromycin, paclitaxel (taxol), vinblastine, vincristine, vindesine, and vinorelbine. Suitable topoisomerase inhibitors include, but are not limited to, amsacrine, etoposide (VP-16), irinotecan, RFS 2000, teniposide, and topotecan. Non-limiting examples of suitable anti-hormonal agents such as aminoglutethimide, aromatase inhibiting 4(5)-imidazoles, bicalutamide, finasteride, flutamide, goserelin, 4-hydroxytamoxifen, keoxifene, leuprolide, LY117018, mitotane, nilutamide, onapristone, raloxifene, tamoxifen, toremifene, and trilostane. Non-limiting examples of targeted therapeutic agents include a monoclonal antibody such as alemtuzumab, bevacizumab, capecitabine, cetuximab, gemtuzumab, heregulin, rituximab, trastuzumab; a tyrosine kinase inhibitor such as imatinib mesylate; and a growth inhibitory polypeptide such as erythropoietin, interleukins (e.g., IL-1, IL-2, IL-3, IL-6), leukemia inhibitory factor, interferons, thrombopoietin, TNF-α, CD30 ligand, 4-1BB ligand, and Apo-1 ligand.

Those of skill in the art appreciate that pharmaceutically acceptable salts, acids, or derivatives of any of the above listed agents may be included in the combination formulations. The mode of administration of the combination formulation can and will vary depending upon the agents and the condition to be treated. Suitable modes of administration are detailed above in section (II)(b)(i).

### (III) Methods for Inhibiting TLR9 Activation

A further aspect of the present invention provides methods for inhibiting the activation of TLR9. In general, the method comprises contacting *in vitro* a cell expressing TLR9 with a compound of Formulas (I), (II), (III), or a pharmaceutically acceptable salt thereof. The method comprises contacting *in vitro* the cell with a compound selected from the group consisting of I-1, I-2, I-3, I-4, I-6, I-7, I-8, I-12, I-13, I-14, I-21, I-22, I-25, I-26, I-27, I-28, I-29, II-1, II-2, II-4, II-5, II-7, II-10, II-11, II-15, II-19, II-21, II-23, II-25, II-26, II-29, II-35, II-36, II-37, II-38, II-42, II-43, II-60, II-62, II-63, II-71, II-72, II-74, II-76, II-78, II-82, II-86, II-88, II-90, II-91, II-92, II-93, II-95, II-96, II-98, II-99, III-1, III-2, III-3, III-4, and III-5.

| Name | Structure | Name | Structure |
|---|---|---|---|
| I-1 | 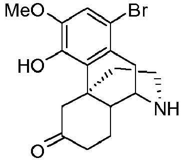 | I-25 | 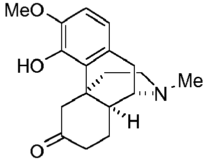 |
| I-28 | 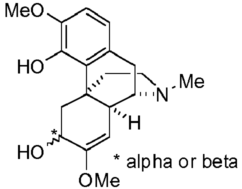 | II-7 | 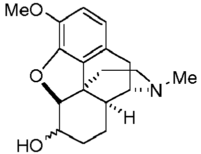 |
| II-38 | 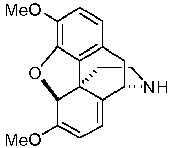 | II-62 | 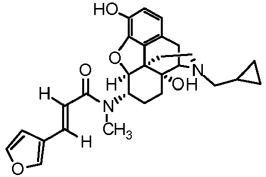 |
| | | II-71 | 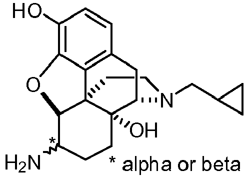 |
| II-63 | 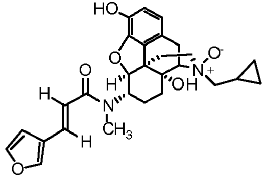 | II-74 | 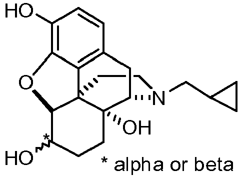 |
| II-76 | 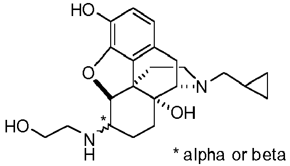 | II-78 | 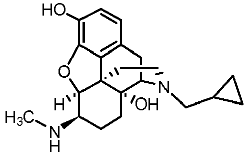 |
| II-82 | 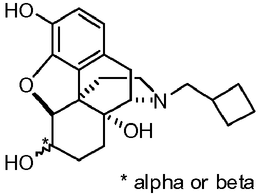 | II-86 | 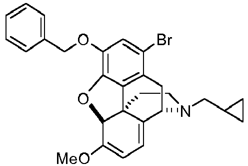 |
| II-88 | 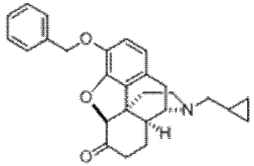 | II-95 | 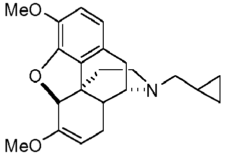 |
| II-96 | 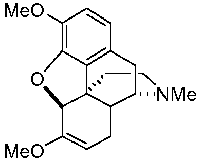 | II-98 | |
| III-1 | 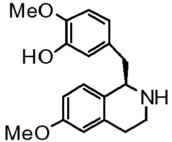 | | |

The method of inhibiting the activation of TLR9 is conducted *in vitro.* Accordingly, the cell expressing TLR9 may be disposed in a subject as detailed above. In preferred embodiments, the cell may be a glial cell, a microglial cell, or an astrocyte. In an exemplary embodiment, the cell may be a glial cell in the central nervous system.

A method for identifying a compound of Formulas (I), (II), or (III) that may be therapeutically effective for treating conditions associated with pain and inflammation is also described. Suitable conditions include traumatic or neuropathic pain, an inflammatory disorder, acetaminophen toxicity, an autoimmune disorder, a neurodegenerative disorder, and cancer. The method comprises determining whether the compound inhibits TLR9 activation. To determine whether the compound of Formulas (I), (II), (III), or a pharmaceutically acceptable salt thereof inhibits TLR9 activation, the method comprises contacting a cell expressing TLR9 with an activation ligand and the compound of interest, wherein TLR9 activation is reduced in the presence of the compound as compared to a control condition in which the cell is contacted with only the activation ligand. Typically, the cell expressing TLR9 that is contacted with the compound of interest is *in vitro.*

Typically, the cell expressing TLR9 will be from a stable cell line. Non-limiting examples of suitable parental cells include HEK293, CHO, BHK, NSO, HDMEC, NHEK, and NHDF cells. The cell line may be HEK293. The cells may be engineered to express TLR9 using standard procedures well known to those of skill in the art. TLR9 may be of mammalian origin, preferably of human origin.

The activation ligand used to activate TLR9 may be methylated DNA, unmethylated DNA, a CpG oligodeoxynucleotide, or an oligodeoxynucleotide. The activation ligand may be CpG oligodeoxynucleotide (ODN) 2006.

The activation of TLR9 in the cell of the in vitro assay may be monitored by measuring the activity of a reporter, wherein the activity of the reporter is coupled to activation of an adaptor protein or kinase that mediates TLR9 signaling by producing intracellular signaling molecules or inducers such as NF-κB or IRF3. Non-limiting examples of suitable reporters include luciferase, alkaline phosphatase, and GFP or other fluorescent proteins. The activation of the reporter may be monitored via luminescence, fluorescence, absorbance, or optical density. The reporter is secreted alkaline phosphatase (SEAP) that is induced by NF-κB, and activation of SEAP is monitored spectrophotometically.

In general, the (+)-morphinan comprising TLR9 antagonist activity may reduce the activation of TLR9 by at least about 10%. The (+)-morphinan may reduce the activation of TLR9 from about 10% to about 15%, from about 15% to about 20%, from about 20% to about 25%, from about 25% to about 30%, from about 30% to about 35%, from about 35% to about 40%, from about 40% to about 45%, from about 45% to about 50%, from about 50% to about 55%, from about 55% to about 60%, from about 60% to about 70%, from about 70% to about 80%, from about 80% to about 90%, or from about 90% to about 99%.

The *in vitro* screening assay may also be used to determine the optimal inhibitory concentration (or IC₅₀) of a (+)-morphinan comprising TLR9 antagonist activity. That is, a dose-response curve may be generated in which the concentration of the (+)-morphinan comprising TLR9 antagonist activity is varied such that the optimal inhibitory concentration may be determined.

### DEFINITIONS

The compounds described herein have asymmetric centers. Compounds of the present invention containing an asymmetrically substituted atom may be isolated in optically active or racemic form. All chiral, diastereomeric, racemic forms and all geometric isomeric forms of a structure are intended, unless the specific stereochemistry or isomeric form is specifically indicated.

The term "acyl," as used herein alone or as part of another group, denotes the moiety formed by removal of the hydroxy group from the group COOH of an organic carboxylic acid, e.g., RC(O)-, wherein R is R¹, R¹O-, R¹R²N-, or R¹S-, R¹ is hydrocarbyl, heterosubstituted hydrocarbyl, or heterocyclo, and R² is hydrogen, hydrocarbyl, or substituted hydrocarbyl.

The term "acyloxy," as used herein alone or as part of another group, denotes an acyl group as described above bonded through an oxygen linkage (O), e.g., RC(O)O-wherein R is as defined in connection with the term "acyl."

The term "alkyl" as used herein describes groups which are preferably lower alkyl containing from one to eight carbon atoms in the principal chain and up to 20 carbon atoms. They may be straight or branched chain or cyclic and include methyl, ethyl, propyl, isopropyl, butyl and hexyl.

The term "alkenyl" as used herein describes groups which are preferably lower alkenyl containing from two to eight carbon atoms in the principal chain and up to 20 carbon atoms. They may be straight or branched chain or cyclic and include ethenyl, propenyl, isopropenyl, butenyl, isobutenyl and hexenyl.

The term "alkynyl" as used herein describes groups which are preferably lower alkynyl containing from two to eight carbon atoms in the principal chain and up to 20 carbon atoms. They may be straight or branched chain and include ethynyl, propynyl, butynyl, isobutynyl and hexynyl.

The term "aromatic" as used herein alone or as part of another group denotes optionally substituted homo- or heterocyclic conjugated planar ring or ring system comprising delocalized electrons. These aromatic groups are preferably monocyclic (e.g., furan or benzene), bicyclic, or tricyclic groups containing from 5 to 14 atoms in the ring portion. The term "aromatic" encompasses "aryl" groups defined below.

The terms "aryl" or "Ar" as used herein alone or as part of another group denote optionally substituted homocyclic aromatic groups, preferably monocyclic or bicyclic groups containing from 6 to 10 carbons in the ring portion, such as phenyl, biphenyl, naphthyl, substituted phenyl, substituted biphenyl, or substituted naphthyl.

The terms "carbocyclo" or "carbocyclic" as used herein alone or as part of another group denote optionally substituted, aromatic or non-aromatic, homocyclic ring or ring system in which all of the atoms in the ring are carbon, with preferably 5 or 6 carbon atoms in each ring. Exemplary substituents include one or more of the following groups: hydrocarbyl, substituted hydrocarbyl, alkyl, alkoxy, acyl, acyloxy, alkenyl, alkenoxy, aryl, aryloxy, amino, amido, acetal, carbamyl, carbocyclo, cyano, ester, ether, halogen, heterocyclo, hydroxy, keto, ketal, phospho, nitro, and thio.

The terms "halogen" or "halo" as used herein alone or as part of another group refer to chlorine, bromine, fluorine, and iodine.

The term "heteroatom" refers to atoms other than carbon and hydrogen.

The term "heteroaromatic" as used herein alone or as part of another group denotes optionally substituted aromatic groups having at least one heteroatom in at least one ring, and preferably 5 or 6 atoms in each ring. The heteroaromatic group preferably has 1 or 2 oxygen atoms and/or 1 to 4 nitrogen atoms in the ring, and is bonded to the remainder of the molecule through a carbon. Exemplary groups include furyl, benzofuryl, oxazolyl, isoxazolyl, oxadiazolyl, benzoxazolyl, benzoxadiazolyl, pyrrolyl, pyrazolyl, imidazolyl, triazolyl, tetrazolyl, pyridyl, pyrimidyl, pyrazinyl, pyridazinyl, indolyl, isoindolyl, indolizinyl, benzimidazolyl, indazolyl, benzotriazolyl, tetrazolopyridazinyl, carbazolyl, purinyl, quinolinyl, isoquinolinyl and imidazopyridyl. Exemplary substituents include one or more of the following groups: hydrocarbyl, substituted hydrocarbyl, alkyl, alkoxy, acyl, acyloxy, alkenyl, alkenoxy, aryl, aryloxy, amino, amido, acetal, carbamyl, carbocyclo, cyano, ester, ether, halogen, heterocyclo, hydroxy, keto, ketal, phospho, nitro, and thio.

The terms "heterocyclo" or "heterocyclic" as used herein alone or as part of another group denote optionally substituted, fully saturated or unsaturated, monocyclic or bicyclic, aromatic or non-aromatic groups having at least one heteroatom in at least one ring, and preferably 5 or 6 atoms in each ring. The heterocyclo group preferably has 1 or 2 oxygen atoms and/or 1 to 4 nitrogen atoms in the ring, and is bonded to the remainder of the molecule through a carbon or heteroatom. Exemplary heterocyclo groups include heteroaromatics as described above. Exemplary substituents include one or more of the following groups: hydrocarbyl, substituted hydrocarbyl, alkyl, alkoxy, acyl, acyloxy, alkenyl, alkenoxy, aryl, aryloxy, amino, amido, acetal, carbamyl, carbocyclo, cyano, ester, ether, halogen, heterocyclo, hydroxy, keto, ketal, phospho, nitro, and thio.

The terms "hydrocarbon" and "hydrocarbyl" as used herein describe organic compounds or radicals consisting exclusively of the elements carbon and hydrogen. These moieties include alkyl, alkenyl, alkynyl, and aryl moieties. These moieties also include alkyl, alkenyl, alkynyl, and aryl moieties substituted with other aliphatic or cyclic hydrocarbon groups, such as alkaryl, alkenaryl and alkynaryl. Unless otherwise indicated, these moieties preferably comprise 1 to 20 carbon atoms.

The "substituted hydrocarbyl" moieties described herein are hydrocarbyl moieties which are substituted with at least one atom other than carbon, including moieties in which a carbon chain atom is substituted with a heteroatom such as nitrogen, oxygen, silicon, phosphorous, boron, or a halogen atom, and moieties in which the carbon chain comprises additional substituents. These substituents include alkyl, alkoxy, acyl, acyloxy, alkenyl, alkenoxy, aryl, aryloxy, amino, amido, acetal, carbamyl, carbocyclo, cyano, ester, ether, halogen, heterocyclo, hydroxy, keto, ketal, phospho, nitro, and thio.

The term "treating," as used herein, refers to inhibiting or alleviating the symptoms of the disease or disorder; reversing, inhibiting, or slowing the progression of the disease or disorder; and/or preventing or delaying the onset of the disease or disorder. The term "treatment", as used herein, unless otherwise indicated, refers to the act of treating as "treating" is defined immediately above.

When introducing elements of the present invention or the preferred embodiments(s) thereof, the articles "a", "an", "the" and "said" are intended to mean that there are one or more of the elements. The terms "comprising", "including" and "having" are intended to be inclusive and mean that there may be additional elements other than the listed elements.

### EXAMPLES

The following examples illustrate various embodiments of the invention.

### Example 1: Toll-Like Receptor Screening

Stimulation of TLRs 2, 3, 4, 5, 7, 8, and 9 was determined by assessing activation of the transcription factor NF-κB in HEK293 cells that were engineered to express the corresponding receptors. Assessment of TLR stimulation was based on the use of an NF-κB-inducible secreted alkaline phosphatase (SEAP) reporter system in which the SEAP reporter was under the control of a promoter inducible by NF-κB. Thus, the degree of activation of TLRs can be indirectly quantified spectrophotometrically by measuring the amount of the SEAP reporter that is produced.

*General procedure.* The appropriate TLR-expressing cells were plated in Growth Medium in a 96-well plate (25,000-50,000 cells/well). The cells were stimulated with the appropriate positive control ligand or no ligand was added (negative control). The positive control ligands were: HKLM was used to stimulate TLR2; poly(I:C) was used to stimulate TLR3; LPS was used to stimulate TLR4; Flagellin was used to stimulate TLR5; CL097 was used to stimulate TLR7; CL075 was used to stimulate TLR8; CpG oligodeoxynucleotide (ODN) 2006 was used to stimulate TLR9. To test whether (+)-morphinans could block that activation of the TLR, the cells were pretreated with an antagonist for 30 minutes prior to addition of the positive control ligand. For this, 20 µL of the stock test compound solution (100 µM in H₂O) was added to give a total volume of 200 µL. The antagonists tested were (+)-naloxone, (+)-naltrexone, sinomenine, and dihydrosinomenine; the final concentration of each was 10 µM. After a 16-20 hr incubation period at 37°C in a CO₂ incubator, 20 µL of the cell culture supernatant was added to 180 µL of QUANTI-Blue™ Media (InvivoGen, San Diego, CA), and the resulting solutions were incubated at 37°C for an additional 1-3 hours according to the manufacturer's instructions. The OD's of the samples were then read at 650 nm on a Beckman Coulter AD 340C Absorbance Detector.

*Results.* The results of the antagonist screening experiments are presented in Table 1 and FIG 1. Each antagonist inhibited the activity of the TLRs. The greatest inhibition, however, was observed with TLR9 (see FIG. 1G). These data indicate that TLR9 is the primary target of (+)-morphinans.

**Table 1. Antagonist Screening**

| | **Average % Inhibition at Toll-Like Receptors** | | | | | | |
|---|---|---|---|---|---|---|---|
| **Compound** | **TLR2** | **TLR3** | **TLR4** | **TLR5** | **TLR7** | **TLR8** | **TLR9** |
| **(+) - Naloxone** | 24 | 27 | 25 | 17 | 18 | 24 | 45 |
| **(+) - Naltrexone** | 21 | 22 | 24 | 22 | 16 | 19 | 51 |
| **Sinomenine** | 30 | 28 | 24 | 19 | 20 | 20 | 55 |
| **Dihydrosinomenine** | 27 | 40 | 23 | 16 | 11 | 21 | 42 |

### Example 2: TLR9 Screening of (+)-Morphinan Library

The secreted alkaline phosphatase reporter is under the control of a promoter inducible by the transcription factor NF-κB. A library of more than 100 (+)-morphinan compounds was screened for TLR9 antagonist activity by assessing NF-κB activation in the HEK293 cells expressing TLR9. This reporter gene allows the monitoring of signaling through the TLR, based on the activation of NF-κB.

In a 96-well plate (200 µL total volume) containing 50,000 cells/well, 20 µL of each sample compound was added to wells, in triplicate, followed by a 30 minute incubation at 37°C and 5% CO₂. After the 30 minutes of incubation, 20 µL of the activator ODN2006 was added to each well. The media added to the wells was designed for the detection of NF-κB induced SEAP expression. After a 16-20 hr incubation period, the OD at 650 nm was read on a Beckman Coulter AD 340C Absorbance Detector. The results of the experiments are shown in Table 2. All compounds were tested at concentrations of 10 µM and 100 nM.

| **Table 2.** TLR9 Inhibition | | | | | |
|---|---|---|---|---|---|
| **Compound ID** | **Chemical Structure** | **OD 650 nm (mean ± sd)** | **% Inhibition* (mean)** | **OD 650 nm (mean ± sd)** | **% Inhibition* (mean)** |
| | | **10 µM** | | **100 nM** | |
| **II-64 (Reference)** | 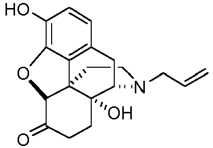 | 0.98 ± 0.01 | 31 | 1.31. ± 0.14 | 8 |
| **II-73 (Reference)** | 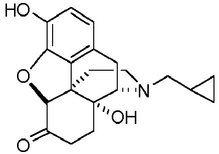 | 0.96± 0.08 | 32 | 1.35 ± 0.13 | 5 |
| **I-30 (Reference)** | 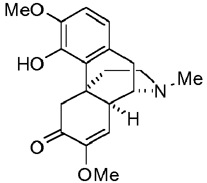 | 0.94 ± 0.05 | 33 | 1.25 ± 0.11 | 12 |
| **I-9 (Reference)** | 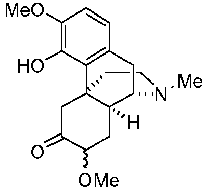 | 1.03 ± 0.01 | 28 | 1.50 ± 0.10 | -6 |
| **I-25** | 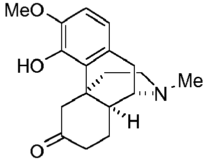 | 0.52 ± 0.07 | 63 | 1.08 ± 0.05 | 24 |
| **I-18 (Reference)** | 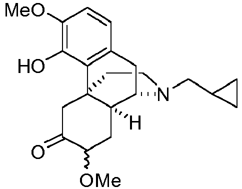 | 1.01 ± 0.06 | 29 | 1.40 ± 0.14 | 1 |
| **II-26** | 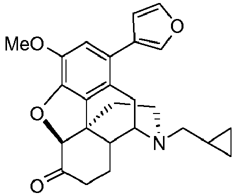 | 0.12 ±0.00 | 92 | 1.08 ± 0.06 | 24 |
| **II-24 (Reference)** | 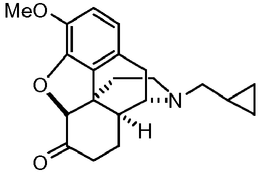 | 0.09 ± 0.01 | 94 | 0.65 ± 0.04 | 54 |
| **II-25** | 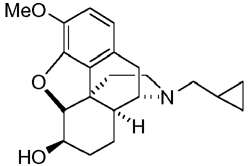 | 0.21 ± 0.06 | 85 | 1.33 ± 0.07 | 6 |
| **II-25** | 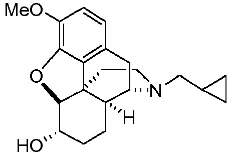 | 0.39 ± 0.01 | 72 | 1.13 ± 0.10 | 21 |
| **II-28** | 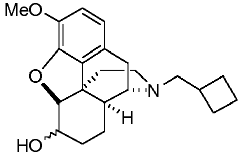 | 0.87 ± 0.03 | 39 | 1.32 ± 0.08 | 7 |
| **II-29** | 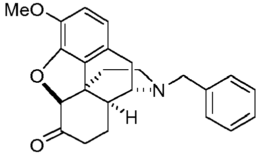 | 0.33 ± 0.03 | 77 | 1.36 ± 0.17 | 4 |
| **II-31** | 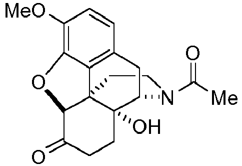 | 1.37 ± 0.11 | 3 | 1.40 ± 0.04 | 2 |
| **II-57** | 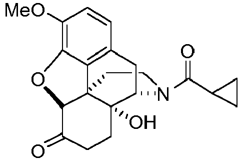 | 1.15 ± 0.03 | 19 | 0.84 ± 0.02 | 41 |
| **II-59** | 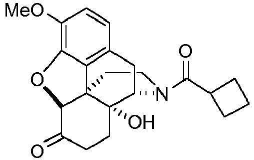 | 1.41 ± 0.13 | 1 | 1.19 ± 0.07 | 17 |
| **II-60** | 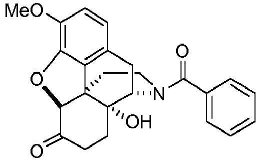 | 0.17 ± 0.03 | 88 | 1.24 ± 0.04 | 13 |
| **II-83** | 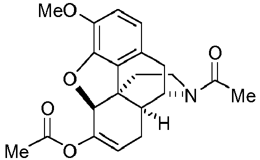 | 1.11 ± 0.09 | 22 | 1.40 ± 0.13 | 1 |
| **II-84 (Reference)** | 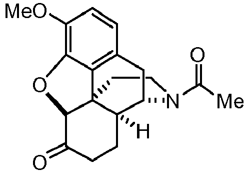 | 1.51 ± 0.08 | -6 | 1.44 ± 0.06 | -1 |
| **II-85 (Reference)** | 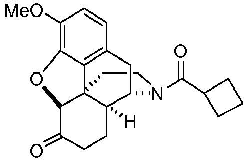 | 1.45 ± 0.04 | -2 | 1.45 ± 0.04 | -2 |
| **II-30** | 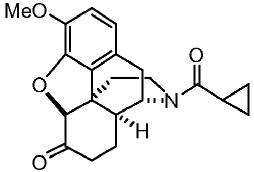 | 1.13 ± 0.06 | 20 | 1.34 ± 0.11 | 5 |
| **II-33** | 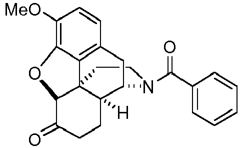 | 1.37 ± 0.03 | 3 | 1.35 ± 0.15 | 5 |
| **II-86** | 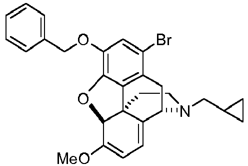 | 0.13 ± 0.03 | 91 | 1.15 ± 0.11 | 19 |
| | | | | | |
| **II**-**88** | 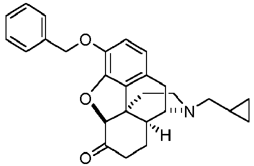 | 0.13 ±0.01 | 91 | 1.12 ± 0.15 | 21 |
| **II-34 (Reference)** | 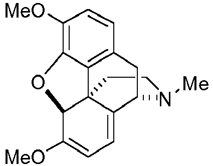 | 0.94 ± 0.08 | 34 | 1.39 ± 0.05 | 2 |
| **II-22** | 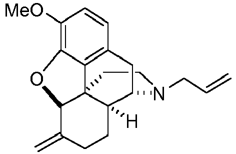 | 0.75 ± 0.06 | 47 | 1.31 ± 0.19 | 8 |
| **II-89** | 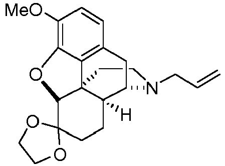 | 1.08 ± 0.02 | 24 | 1.53 ± 0.10 | -8 |
| **I-26** | 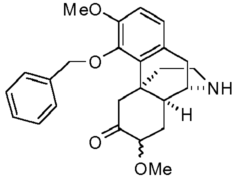 | 0.19 ± 0.02 | 87 | 1.15 ± 0.03 | 19 |
| **II-70** | 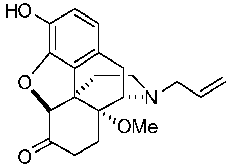 | 0.79 ± 0.03 | 45 | 1.12 ± 0.04 | 21 |
| **II-79** | 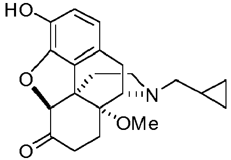 | 1.06 ± 0.11 | 25 | 1.07 ± 0.03 | 25 |
| **III-2** | 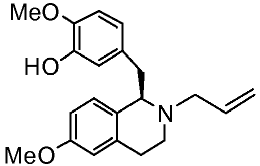 | 0.42 ± 0.04 | 71 | 1.13 ± 0.02 | 21 |
| **III-3** | 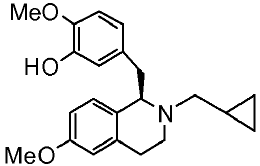 | 0.13 ± 0.01 | 91 | 1.03 ± 0.03 | 27 |
| **III-4** | 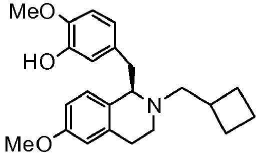 | 0.11 ± 0.01 | 92 | 1.04 ± 0.04 | 27 |
| **III-5** | 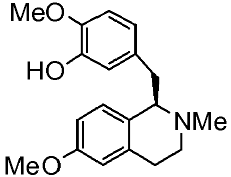 | 0.29 ± 0.01 | 80 | 1.11 ± 0.02 | 22 |
| **II-35** | 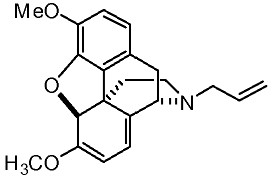 | 0.34 ± 0.03 | 76 | 1.04 ± 0.08 | 27 |
| **II-36** | 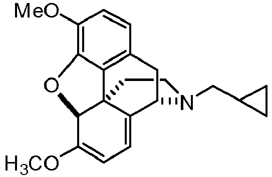 | 0.14 ± 0.01 | 90 | 1.14 ± 0.02 | 20 |
| **II-37** | 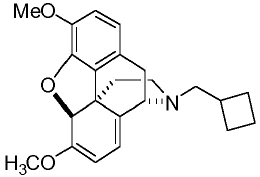 | 0.18 ±0.06 | 87 | 1.13 ± 0.07 | 20 |
| **II-38** | 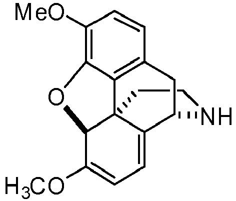 | 0.12 ± 0.01 | 92 | 0.86 ± 0.01 | 40 |
| **I-22** | 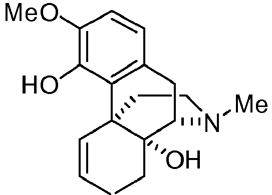 | 0.10 ± 0.01 | 93 | 0.93 ± 0.15 | 34 |
| **II-41** | 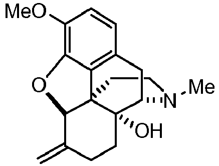 | 1.02 ± 0.09 | 28 | 1.27 ± 0.03 | 11 |
| **II-42** | 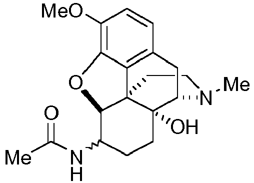 | 0.14 ± 0.02 | 90 | 1.00 ± 0.03 | 29 |
| **II-43** | 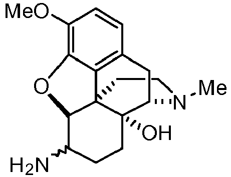 | 0.09 ± 0.00 | 94 | 0.10 ± 0.01 | 93 |
| **II-44** | 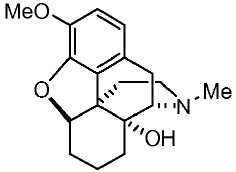 | 1.03 ± 0.12 | 27 | 1.18 ± 0.02 | 17 |
| **II-75** | 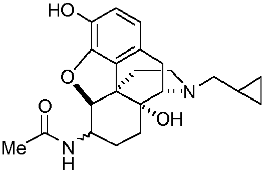 | 0.91 ± 0.11 | 36 | 1.08 ± 0.02 | 24 |
| **II-76** | 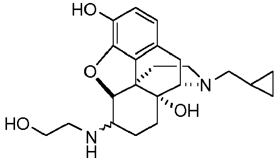 | 0.10 ± 0.01 | 93 | 0.16 ± 0.01 | 89 |
| **II-77 (Reference)** | 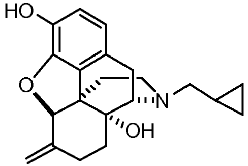 | 0.98 ± 0.02 | 37 | 1.24 ± 0.04 | 13 |
| **II-71** | 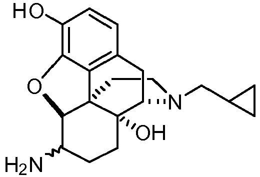 | 0.10 ± 0.00 | 93 | 0.19 ± 0.01 | 87 |
| **II-72** | 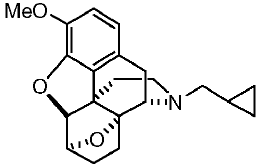 | 0.49 ± 0.01 | 65 | 1.10 ± 0.02 | 22 |
| **II-40 (Reference)** | 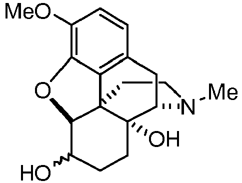 | 1.37 ± 0.07 | 4 | 1.41 ± 0.13 | 0 |
| **II-46** | 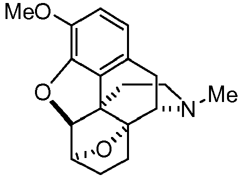 | 1.30 ± 0.02 | 8 | 1.24 ± 0.02 | 13 |
| **II-47 (Reference)** | 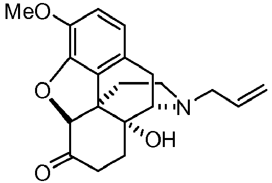 | 0.78 ± 0.07 | 45 | 1.18 ± 0.13 | 17 |
| **II-48** | 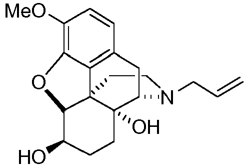 | 1.23 ± 0.02 | 13 | 1.43 ± 0.05 | 0 |
| **II-48** | 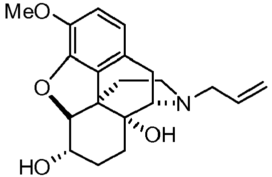 | 1.06 ± 0.02 | 25 | 1.21 ± 0.02 | 15 |
| **II-51** | 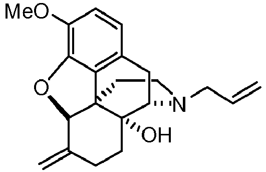 | 0.84 ± 0.05 | 41 | 1.20 ± 0.04 | 16 |
| **II-55** | 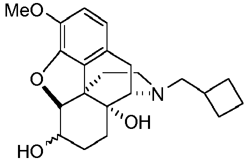 | 1.17 ± 0.08 | 18 | 1.46 ± 0.08 | -3 |
| **II-74** | 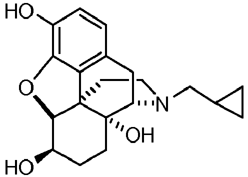 | 0.12 ± 0.00 | 92 | 0.61 ± 0.03 | 57 |
| **II-90** | 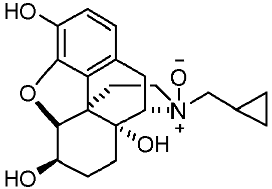 | 0.17 ±0.05 | 88 | 1.39 ± 0.03 | 2 |
| **II-78** | 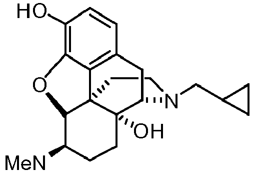 | 0.11 ± 0.01 | 92 | 0.14 ± 0.02 | 90 |
| **II-82** | 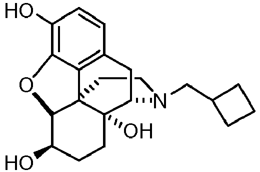 | 0.49 ± 0.03 | 65 | 1.37 ± 0.02 | 4 |
| **II-62** | 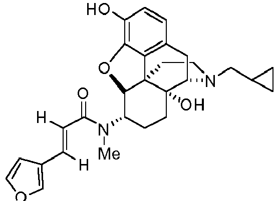 | 0.18 ± 0.01 | 87 | 1.34 ± 0.05 | 6 |
| **II-63** | 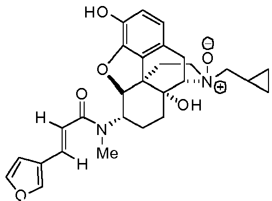 | 0.13 ± 0.01 | 91 | 0.23 ± 0.07 | 84 |
| **I-2** | 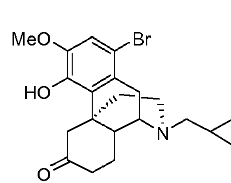 | 0.14 ± 0.02 | 90 | 1.10 ± 0.06 | 22 |
| **I-1** | 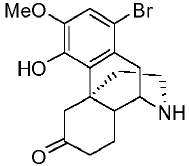 | 0.21 ± 0.01 | 85 | 1.18 ± 0.10 | 17 |
| **I-3** | 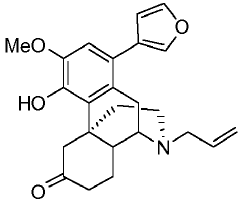 | 0.11 ± 0.00 | 92 | 1.12 ± 0.01 | 21 |
| **I-7** | 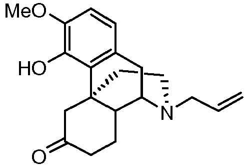 | 0.16 ± 0.01 | 89 | 1.14 ± 0.03 | 20 |
| **II-1** | 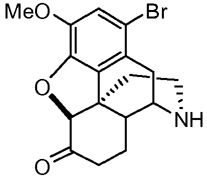 | 0.19 ±-.02 | 86 | 0.97 ± 0.04 | 32 |
| **II-2** | 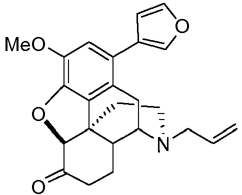 | 0.12 ± 0.01 | 92 | 1.23 ± 0.05 | 13 |
| **II-4** | 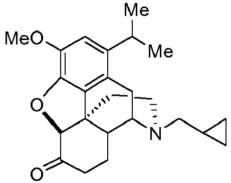 | 0.11 ± 0.01 | 92 | 1.18 ± 0.06 | 17 |
| **I-8** | 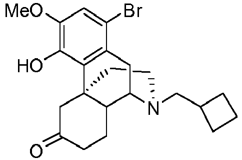 | 0.11 ± 0.01 | 92 | 1.13 ± 0.07 | 20 |
| **I-11 (Reference)** | 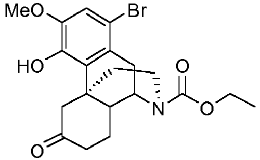 | 1.98 ± 0.09 | -39 | 1.49 ± 0.04 | -5 |
| **I-12** | 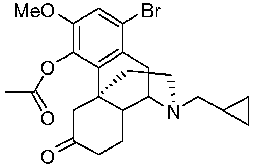 | 0.13 ± 0.01 | 91 | 1.19 ± 0.07 | 16 |
| **I-13** | 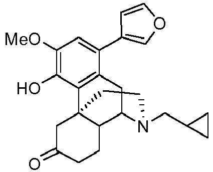 | 0.13 ± 0.01 | 91 | 1.34 ± 0.12 | 6 |
| **I-27** | 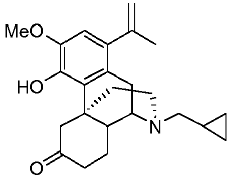 | 0.10 ± 0.00 | 93 | 1.13 ± 0.04 | 20 |
| **II-15** | 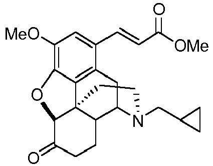 | 0.23 ± 0.03 | 84 | 1.22 ± 0.06 | 14 |
| **II-16** | 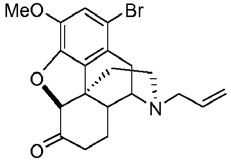 | 1.24 ± 0.11 | 13 | 1.43 ± 0.01 | -1 |
| **II-23** | 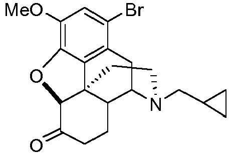 | 0.53 ± 0.05 | 63 | 1.16 ± 0.01 | 18 |
| **I-21** | 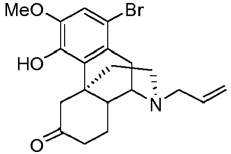 | 0.23 ± 0.01 | 84 | 1.40 ± 0.02 | 2 |
| **I-5 (Reference)** | 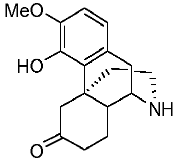 | 0.87 ± 0.04 | 38 | 1.31 ± 0.09 | 8 |
| **I-4** | 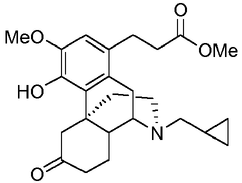 | 0.46 ± 0.05 | 68 | 1.17 ± 0.06 | 18 |
| **I-6** | 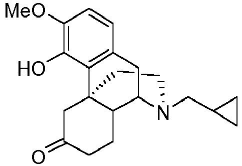 | 0.12 ± 0.02 | 91 | 1.29 ± 0.07 | 9 |
| **II-5** | 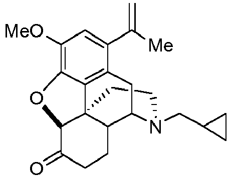 | 0.14 ± 0.00 | 90 | 1.11 ± 0.03 | 22 |
| **II-11** | 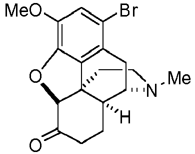 | 0.44 ± 0.05 | 69 | 1.22 ± 0.10 | 14 |
| **I-14** | 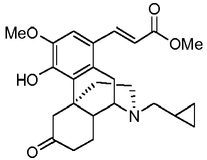 | 0.13 ± 0.00 | 91 | 1.18 ± 0.05 | 17 |
| **II-7** | 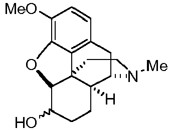 | 0.13 ± 0.02 | 91 | 1.32 ± 0.08 | 7 |
| **II-91** | 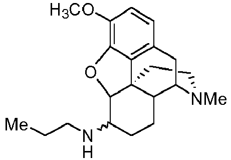 | 0.09 ± 0.01 | 93 | 0.12 ± 0.01 | 92 |
| **II-92** | 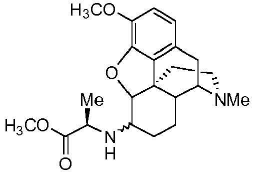 | 0.73 ± 0.06 | 48 | 1.09 ± 0.03 | 23 |
| **II-93** | 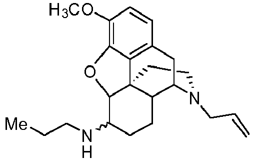 | 0.11 ± 0.01 | 92 | 0.12 ± 0.00 | 91 |
| **II-21** | 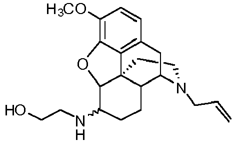 | 0.09 ± 0.01 | 93 | 0.19 ± 0.02 | 87 |
| **II-94** | 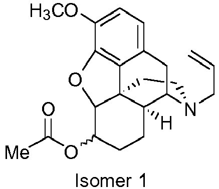 | 1.16 ± 0.08 | 18 | 1.13 ± 0.04 | 20 |
| **II-94** | 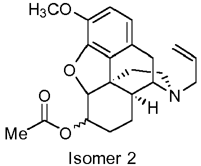 | 1.30 ± 0.05 | 8 | 1.17 ± 0.03 | 17 |
| **II-9** | 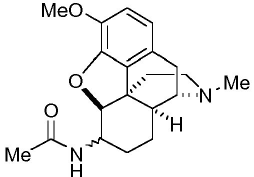 | 1.00 ± 0.02 | 29 | 1.25 ± 0.09 | 12 |
| **II-20** | 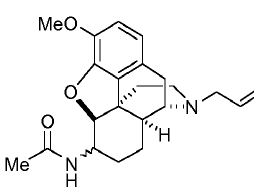 | 1.07 ± 0.04 | 25 | 1.29 ± 0.02 | 9 |
| **II-19** | 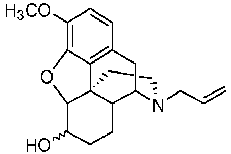 | 0.15 ± 0.01 | 90 | 1.19 ± 0.03 | 16 |
| **II**-**8** | 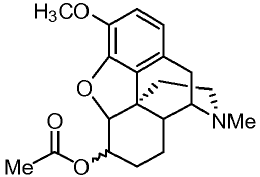 | 1.33 ± 0.06 | 7 | 1.15 ± 0.07 | 19 |
| **II-53** | 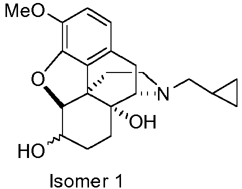 | 1.32 ± 0.13 | 7 | 1.22 ± 0.05 | 14 |
| **II-53** | 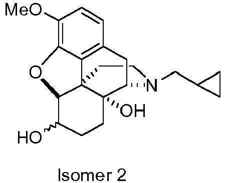 | 1.00 ± 0.03 | 29 | 1.41 ± 0.09 | 1 |
| **II-74** | 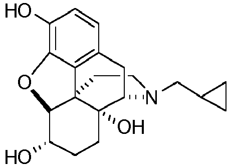 | 1.26 ± 0.06 | 11 | 1.19 ± 0.06 | 16 |
| **II-95** | 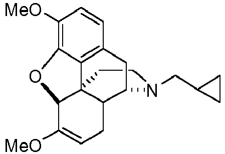 | 0.37 ± 0.03 | 74 | 1.11 ± 0.04 | 22 |
| **II-96** | 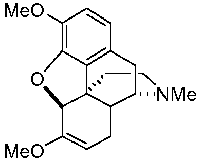 | 0.42 ± 0.02 | 71 | 1.15 ± 0.06 | 19 |
| **II-97** | 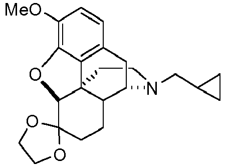 | 1.42 ± 0.07 | 0 | 1.22 ± 0.02 | 14 |
| **II-98** | 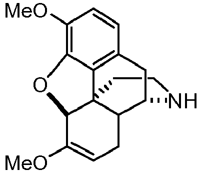 | 0.56 ± 0.03 | 60 | 1.02 ± 0.02 | 28 |
| **II-99** | 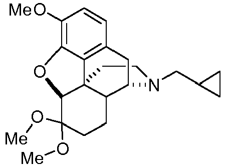 | 0.14 ± 0.01 | 90 | 1.32 ± 0.00 | 7 |
| **II-100** | 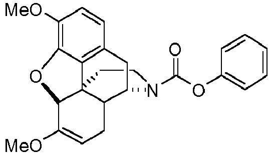 | 1.45 ± 0.11 | -2 | 1.16 ± 0.02 | 18 |
| **II-12 (Reference)** | 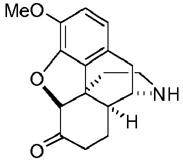 | 0.81 ± 0.03 | 43 | 1.12 ± 0.08 | 21 |
| **II-101 (Reference)** | 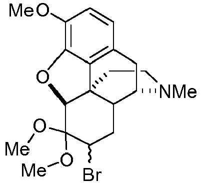 | 0.94 ± 0.02 | 34 | 1.35 ± 0.06 | 5 |
| **III-1** | 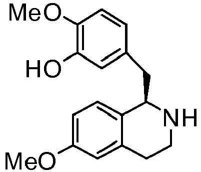 | 0.67 ± 0.01 | 53 | 1.34 ± 0.06 | 6 |
| **II-39 (Reference)** | 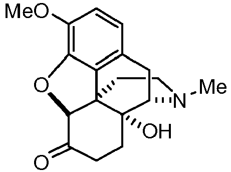 | 1.06 ± 0.06 | 25 | 1.23 ± 0.03 | 14 |
| **II-10** | 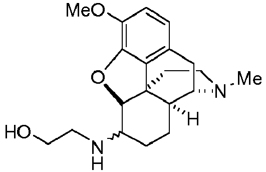 | 0.10 ± 0.01 | 93 | 0.24 ± 0.02 | 83 |
| **I-16 (Reference)** | 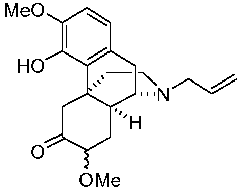 | 0.91 ± 0.04 | 36 | 1.50 ± 0.01 | -5 |
| **I-28** | 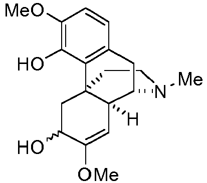 | 0.12 ± 0.01 | 91 | 1.24 ± 0.02 | 13 |
| **I-10 (Reference)** | 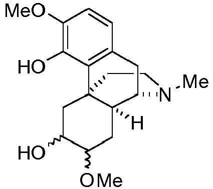 | 1.04 ± 0.08 | 27 | 1.32 ± 0.04 | 7 |
| **I-29** | 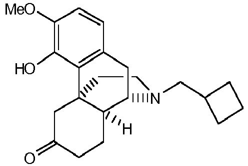 | 0.11 ± 0.00 | 92 | 1.06 ± 0.03 | 25 |
| **II-61 (Reference)** | 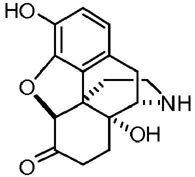 | 1.22 ± 0.07 | 14 | 1.23 ± 0.02 | 13 |
| * Control (no activator) = 0.14 OD 650 nm (*n* = 12); Activator (ODN2006) = 1.42 OD 650 nm (*n* = 12) | | | | | |

This screening experiment identified those (+)-morphinan compounds comprising the greatest TLR9 antagonist activity. Even at a concentration as low as 100 nM, some compounds inhibited TLR9 in excess of 90%.

### Example 3: Analgesic Assessment of (+)-Naloxone on Mechanical Allodynia

Neuropathic pain affects approximately 1% of the U.S. population and is extremely difficult to manage. Usually the pain is chronic, severe, and fails to respond to traditional analgesic drugs. Fortunately, one of the most widely used animal models for neuropathic pain closely mimics the pain endured by patients. In this model, termed the chronic constriction injury (CCI) or Bennett model, four closely spaced ligatures tied loosely around the sciatic nerve of a rat cause demyelination of the nerve, resulting in spontaneous pain, such as prolonged paw elevation and licking of the ligated paw. Induced pain in the nerve-injured limb can be measured using Von Frey filaments, applied to the plantar surface of the paw to test for responses to non-noxious tactile stimulation. The onset of this heightened mechanical allodynia is quite rapid and it persists for 2-3 months.

In this example, the CCI ligation was performed on the left sciatic nerve in three groups of rats. Von Frey testing of mechanical allodynia was performed on both paws on Day 14 to determine the analgesic efficacy of the test agent. The testing was performed pre-dosing, and at 30 and 90 minutes post-dosing.

*Animals.* A total of thirty-four (34) male Sprague-Dawley rats were ordered from Harlan Sprague-Dawley. The animals were specific pathogen free and weighed approximately 175 - 200 grams upon arrival. A visual health inspection was performed on each animal to include evaluation of the coat, extremities and abnormal signs in posture or movement. Animals were individually identified with a unique ear tag assigned at receipt. The animals were individually housed in clear polycarbonate plastic cages and received enrichment in the way of Enrich-o-cobs bedding. Cage cards were affixed to their cages that identified study number, animal number, treatment designation, species/strain, and gender. The animals were acclimated for 5 days prior to the commencement of the experimental procedures. The room number in which the animals were housed throughout the study period was detailed in the study records. The temperature was maintained at 18-26°C (64-79°F) with a relative humidity of 30-70%. Temperature and humidity were monitored and daily minimums and maximums recorded.

*Treatment groups.* The animals were allocated to treatment groups based on their baseline Von Frey data, measured prior to surgery. The mechanical allodynia scores for each group were reviewed to ensure that the mean values and standard deviation satisfied the assumption of homogeneity. Table 3 presents the treatment groups. Thirty-one (31) animals were used on the study. Thirty (30) animals were initially allocated to treatment groups, and the remaining four (4) animals were held as spares. One spare was then used to replace an animal that died during surgery. Body weights were taken one day after arrival, prior to surgery and weekly thereafter. On Day 14, after the final behavioral testing, the animals were euthanized by carbon dioxide asphyxiation. No necropsy was performed nor tissues collected.

**Table 3. Treatment Groups**

| **Group** | **Description** | **Test Article** | **Route/ Frequency** | **Dose mg/kg** | **#/Group** |
|---|---|---|---|---|---|
| 1 | Bennett surgery | Vehicle | S.C. injection | 0 | 10 |
| 2 | Bennett surgery | Gabapentin | I.P. injection | 100 mg/kg | 10 |
| 3 | Bennett surgery | (+)-Naloxone·HCl | S.C. injection | 66.7 mg/kg | 10 |

*Surgery.* All surgeries were performed under aseptic conditions. Prior to surgery, the rats were sedated using inhaled Isoflurane anesthetic. The left leg was shaved and prepped. The common sciatic nerve was exposed and freed from adherent tissue at mid-thigh by separating the muscle (biceps femoris) by blunt dissection. Proximal to the sciatic nerve's trifurcation; approximately 7 mm of nerve was freed from the adhering tissue. Four ligatures, approximately 1 mm apart, were tied loosely around the nerve using 6.0 chromic catgut. Each of the sutures was tied loosely with a square knot around the sciatic nerve. A brief twitch in the muscle surrounding the exposure was an indicator of the desired degree of constriction. The site was then closed using the appropriate suture material. Post-operative care and observations were carried out until the animal recovered consciousness. Animals were observed for dragging (from inadvertent surgical damage) on Days 1 & 3 post-operatively and daily for signs of ill health and general well being.

*Behavioral testing.* The animals underwent a series of behavioral testing for neuropathic pain assessment. A Von Frey test of mechanical allodynia was performed on both hind paws prior to surgery to achieve a baseline measurement for randomization and then following surgery on Day 14 (test article efficacy) according to Table 4.

**Table 4. Behavioral Testing Schedule**

| **Day** | **Time point** |
|---|---|
| 0 | Baseline (for randomization) |
| 14 | Pre-dose |
| 14 | 30 minutes post-dose |
| 14 | 90 minutes post-dose |

*Mechanical allodynia.* Twice prior to surgery, the animals were acclimated to the allodynia apparatus. This habituated the rats to the testing devices to familiarize them with the apparatus so they were calm at the time of testing. The test for mechanical allodynia was used to assess the anti-nociceptive properties of analgesic compounds. Animals were first habituated to the testing chamber so that they were calm enough for their pain threshold to be assessed. A technician blind to the treatment groups applied light pressure to both hindpaws of the rat using a series of graded nylon filaments (Von Frey filaments) of increasing diameter. The filaments were pressed perpendicularly against the ventral surface of the paw until they bent and the rat responded by withdrawing its paw when this was considered painful. Threshold allodynia was determined using the Chaplan up-down method which provided the precise force for withdrawal for each rat using a psychophysical scale of testing. Both the left and right hindpaws were tested at each time point. The order of testing was the ipsilateral (affected) limb followed by the contralateral limb. There were approximately 20 minutes in between testing the two limbs.

*Dosing.* On Day 14, animals in Groups 1 and 3 received an S.C. injection of either vehicle or (+)-naloxone·HCl according to Table 3. They were dosed at 1 mL/kg. On Day 14, animals in Group 2 received an I.P. injection of Gabapentin according to Table 3. They were also dosed at 1 mL/kg.

*Statistics.* The allodynia data were compared among groups and between paws and time points with a 3 x 2 ANOVA. When data were significant (p<0.05), a Bonferroni post-hoc test was applied to determine individual group differences.

*Results.* The mechanical allodynia testing was performed prior to surgery (baseline), and at Day 14 post-surgery at pre-dosing, and 30 and 90 min post-dosing. The baseline data are not included in the figures, but all animals achieved the maximal score of 17 g, indicating no sensitivity to the highest force of filament used in this test. At pre-dose testing on Day 14, there were some animals that continued to respond with a score of 17 g. This indicated that the surgery had not been effective at causing neuropathic pain in this subset of animals, and therefore, they were removed from the data set, and excluded from statistical analyses. The final sample sizes and the allodynia data are presented in FIG. 2A for the left paw (affected) and FIG. 2B (unaffected) for the right paw.

The data in FIG. 2A demonstrate a significant allodynia in the left paw for all three groups in relation to the pre-surgery baseline data (p<0.01 for treatment, p<0.001 for time). After dosing, both Gabapentin and (+)-naloxone were effective at significantly reducing the allodynia at both 30 and 90 min. The right paw data are depicted in FIG. 2B and these data reveal that there was no allodynia in the right paw as expected in this model. There were no significant group differences or differences across time with the exception of the Gabapentin group, which demonstrated a higher threshold for mechanical allodynia testing at the 90 min timepoint than was observed in the vehicle group. This is not surprising, given that Gabapentin was administered peripherally and has a robust analgesic effect bilaterally.

*Conclusions.* This study examined the efficacy of the test agent, (+)-naloxone, to reduce allodynia in the left paw of animals that achieved neuropathic pain when tested on Day 14 post-surgery. The results showed that significant analgesia was achieved by (+)-naloxone at both timepoints tested, which was similar to the results of the positive control, Gabapentin. Thus, (+)-naloxone·HCl was an effective analgesic for reversing neuropathic pain over a timecourse of 30 and 90 min post-dosing on Day 14 using a dose of 66.7 mg/kg.

### Example 4: Evaluation of (+)-Morphinans to Inhibit/Reduce Acetaminophen-Induced Hepatotoxicity

Acetaminophen-induced liver damage is the most common cause of death due to acute liver failure. Treatment with a (+)-morphinan may reduce or prevent acute liver injury induced by acetaminophen (APAP). The effectiveness of the (+)-morphinan compounds disclosed herein to reduce liver injury after exposure to a toxic dose of APAP may be tested in mouse model of APAP-induced toxicity.

For this, APAP-induced liver toxicity may be induced in male C57BL/6 mice aged between 8-10 weeks by a single intraperitoneal injection (ip) of APAP in PBS at a dose of 500 mg/Kg (Imaeda et al. (2009) J. Clin. Invest. 119(2):305-314). The (+)-morphinan may be administered at 10 - 60 mg/Kg as a single injection (subcutaneously (sc) or ip) at the same time as APAP injection. Within this experimental model, the ability of (+)-morphinans to reduce liver toxicity may be tested in the following groups;
1) No APAP and PBS (n= 5)
2) No APAP and (+)-morphinan (n= 5)
3) APAP and PBS (n= 25)
4) APAP and (+)-morphinan (n= 25)

At 12 hrs post-injection, all mice may be sacrificed and the following data collected a) liver histology with H&E staining, b) serum ALT (alanine transaminase), and/or c) whole liver mRNA levels for Pro-IL-1β (pro-interleukin 1 beta).

It is predicted that group 3 will display significant liver injury. Group 4, however, may have significantly less liver injury, indicating the (+)-morphinans protect the liver from damage induced by a toxic dose of APAP.

### Example 5: Evaluation of (+)-Morphinans to Inhibit/Reduce Inflammation Using AIA Model

The anti-inflammatory effects of (+)-morphinans may be tested in model animal systems. For example, the Adjuvant Induced Arthritis (AIA) Lewis rat model is good animal model for rheumatoid arthritis, a disease characterized by a T-lymphocyte and macrophage cellular infiltrate.

Male Lewis rats (7 weeks old; ∼ 200 g) may be injected with 0.1 ml *Mycobacterium butyricum* in Incomplete Freund's Adjuvant subcutaneous in the base of the tail. The rats may be divided into control and treatment groups (∼10 rats/group) as indicated below. The animals may be treated twice daily beginning on the day of injection of mycobacterium and continuing for 4 weeks.
1) Vehicle: 2.5 ml/kg of 20% HBC (2-Hydroxypropyl, β-cyclodextrin)
2) Prednisolone (Positive Control) 4.5 mg/kg, ip, BID
3) (+)-morphinan 5-20 mg/kg, sc, BID

Rats may be weighed twice a week during the course of the trial. Starting the second week, rats may be observed two-three times a week for clinical signs of AIA. For this, all four paws may be examined and scored using the following scale for a maximum of 16 points.
0 = no signs of inflammation
1= moderate redness, first indication of swelling, joint still flexible
2= moderate redness, moderate swelling, joint still flexible
3= redness, significant swelling and distortion of the paw, joint beginning to fuse; tentative in using or putting weight on paw
4= redness, gross swelling and distortion of the paw, joint completely fused; unwilling to use or put weight on paw

At the end of the four week trial, the animals may be euthanized, blood/serum samples may be collected for cytokine profiling, and paws with ankle joints may be preserved for histology.

### Example 6: Evaluation of (+)-Morphinans to Inhibit/Reduce Inflammation Using EAE Model

The effectiveness of (+)-morphinans to reduce inflammation may also be tested in the EAE (Experimental Autoimmune Encephalomyelitis (EAE) Lewis rat model. For this, female Lewis rats (7 weeks old; ∼ 200 g) may be injected with 0.05 ml *Mycobacterium tuberculosis* in a guinea pig spinal cord emulsion into each main foot pad of the rear paws. The rats may be divided into control and treatment groups (∼10 rats/group) as indicated below. The animals may be treated twice daily beginning on the day of injection of mycobacterium.
1) Vehicle: 2.5 ml/kg of 20% HBC
2) Prednisolone (Positive Control) 4.5 mg/kg, ip, BID
3) (+)-morphinan 5-20 mg/kg, sc, BID

Rats may be weighed twice a week and, starting the second week, may be observed two-three times a week for clinical signs of EAE using the scoring system detailed below. Rats which are borderline in scores may be given a one half score, such as 3.5. Moribund rats will be euthanized.

| **EAE Score** | **Symptoms** |
|---|---|
| 0 | Normal |
| 1 | Limp tail |
| 2 | Incomplete paralysis of one or both hind limbs (weakness, limping, shaking) |
| 3 | Complete paralysis of one hind limb or both hind limbs can move but do not help in movement of the body |
| 4 | Complete paralysis of both hind limbs (back half or rat is dragged around the cage) |
| 5 | Complete paralysis of hind limbs and weakness of one or both forelimbs or moribund, or death |

### Example 7: Evaluation of (+)-Morphinans in Combination with Chemotherapeutic Agent to Treat Xenograft Tumor

To determine whether (+)-morphinans increase the efficacy of chemotherapeutic agents, the following trial may be performed. Female nude mice (Hsd:Athymic Nude-Foxn1 nu/nu; 5-6 weeks old) may be injected sc into the right shoulder region with EGFR expressing human tumor cells. The mice may be divided into the following treatment groups (∼10 rats/group):
1) Vehicle: saline
2) Cisplatin 6 mg/kg, iv, 3X daily
3) Cisplatin 6 mg/kg iv and (+)-morphinan 20 mg/kg, sc, 3X daily
4) EGFR inhibitor, iv, 3X daily
5) EGFR inhibitor iv and (+)-morphinan 20 mg/kg, sc, 3X daily

Palpation for tumors may begin 7 days post implantation. Tumors may be observed and measured 3 times a week. Caliper measures - in mm width (small measure) x length (large measure). Body weight may be recorded on day 1 of the trial (day of cell implantation) and once weekly until necropsy. NIH euthanasia guidelines for rodent tumors will be followed (e.g., if tumor diameter exceeds 20 mm, if tumor is ulcerated tumor, if tumor severely restricts the animal's ability to eat, drink, eliminate wastes, breathe, or ambulate, or if animal is becoming emaciated and/or loses more than 20% of pre-study weight).

## Claims

1. A compound chosen from the following formulae: 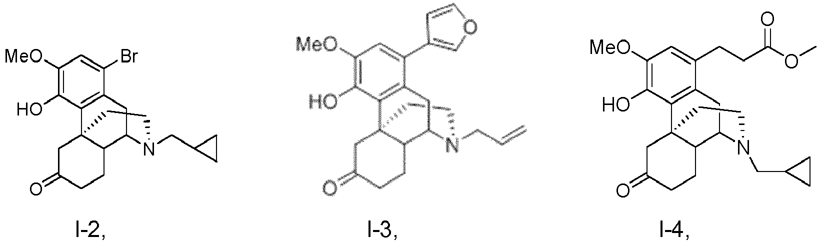 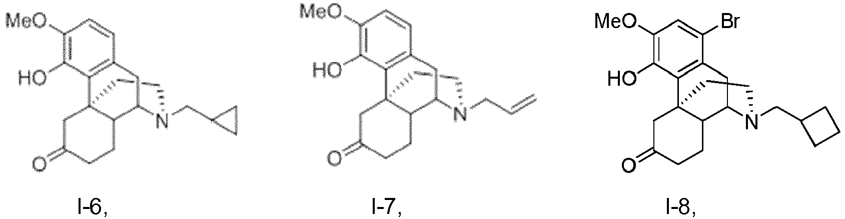 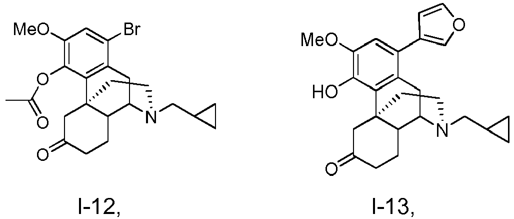 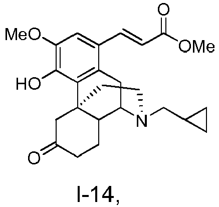 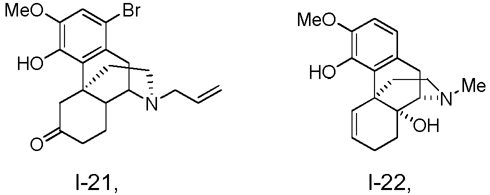 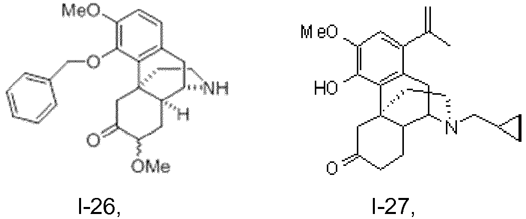 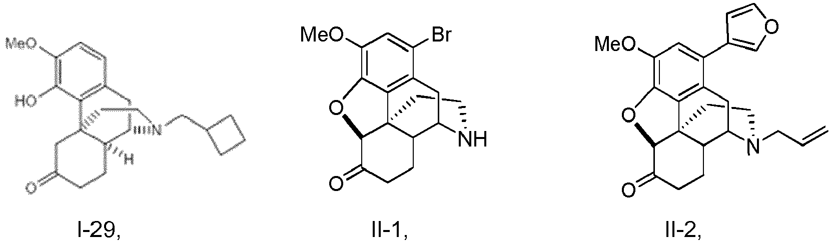 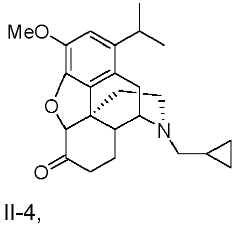 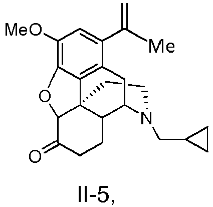 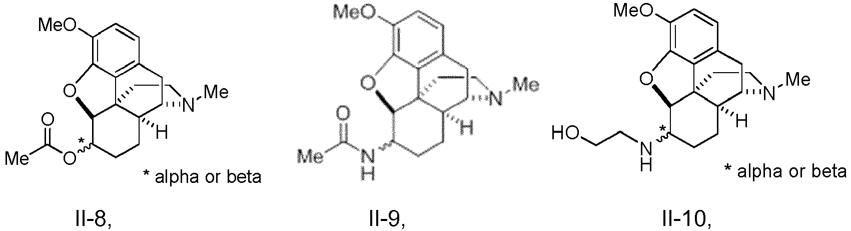 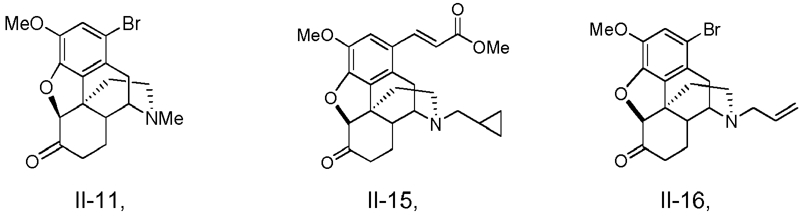 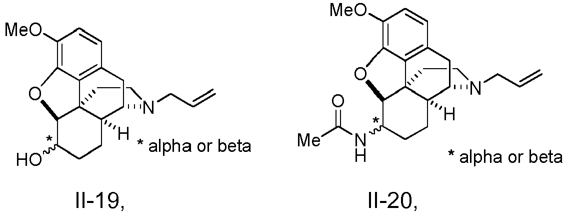 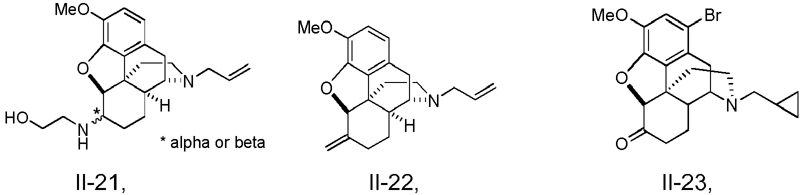 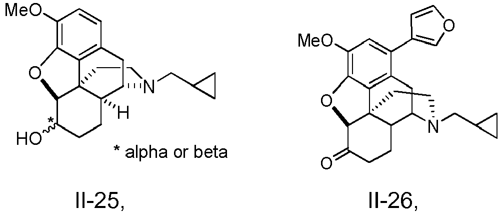 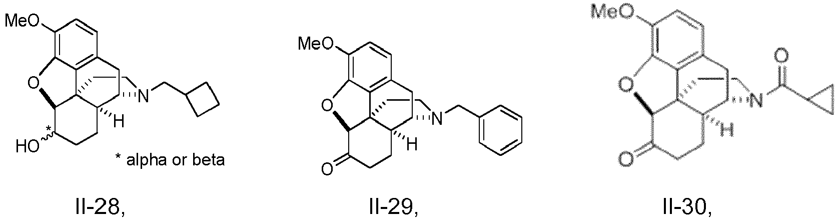 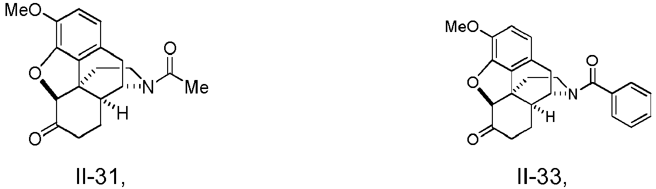 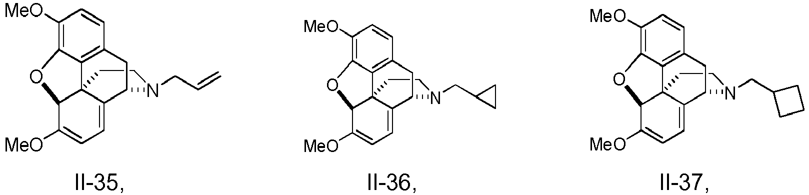 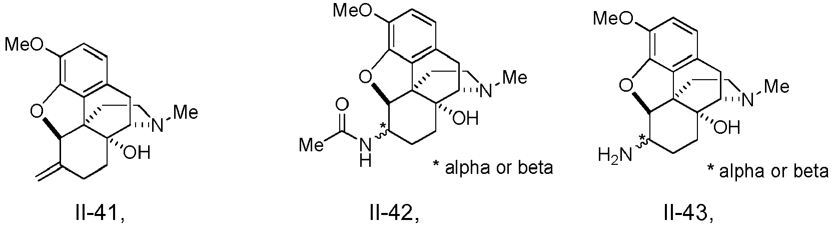 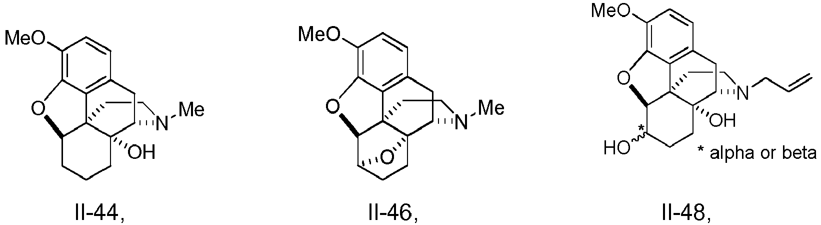 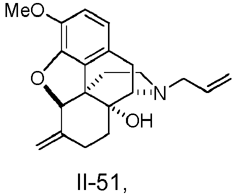 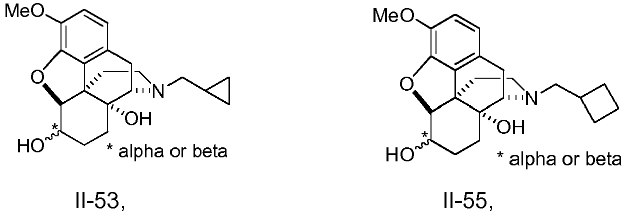 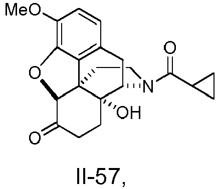 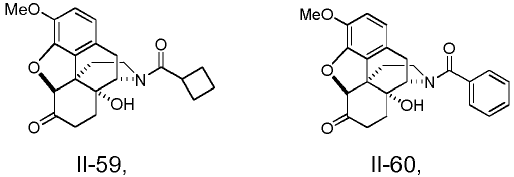 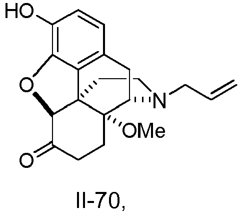 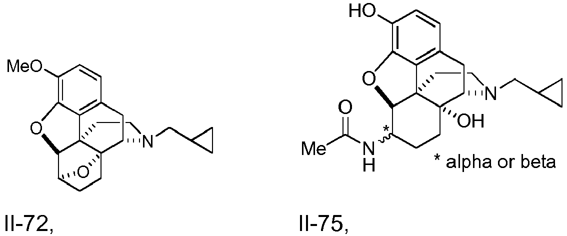 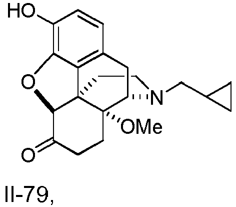 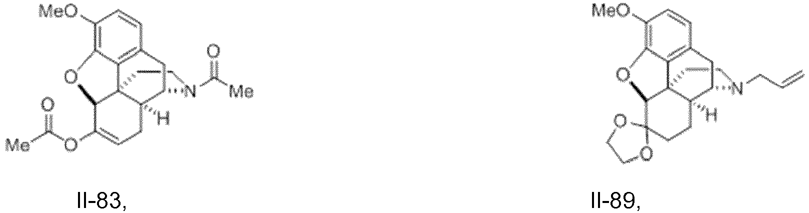 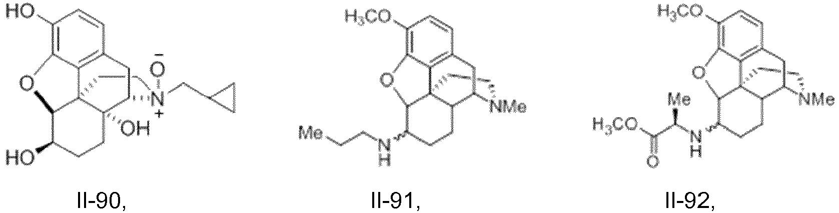 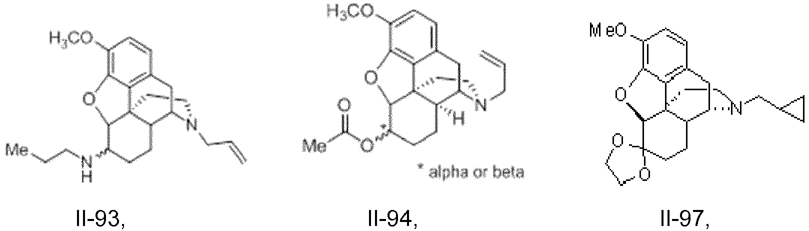 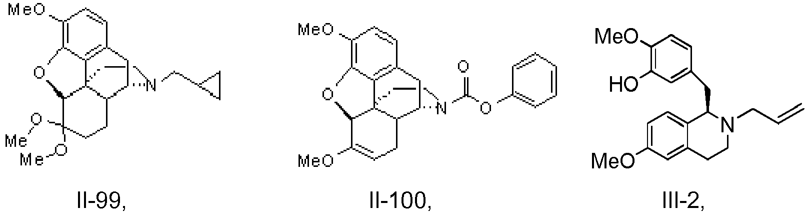 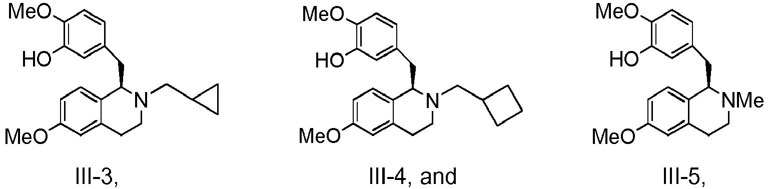 or a pharmaceutically acceptable salt thereof.

2. A method for inhibiting TLR9 activation, the method comprising contacting *in vitro* a cell expressing TLR9 with a compound chosen from the following formulae: 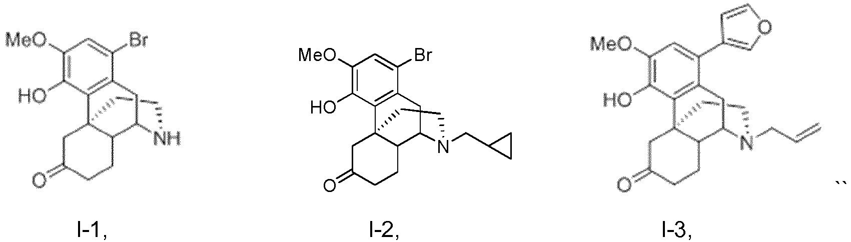 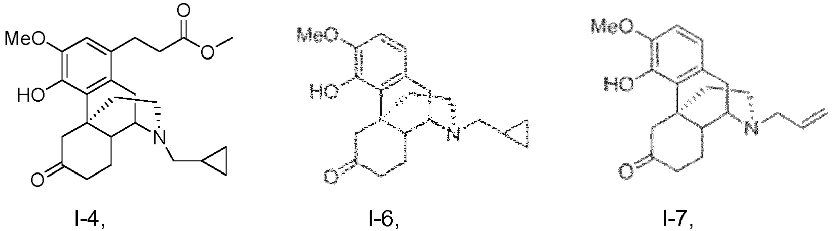 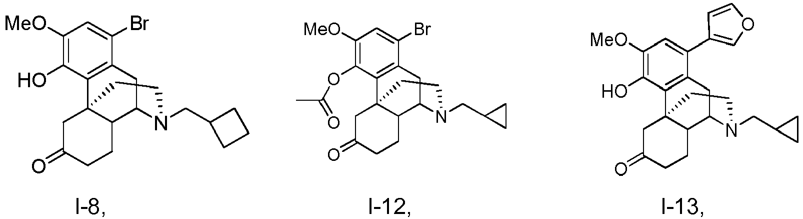 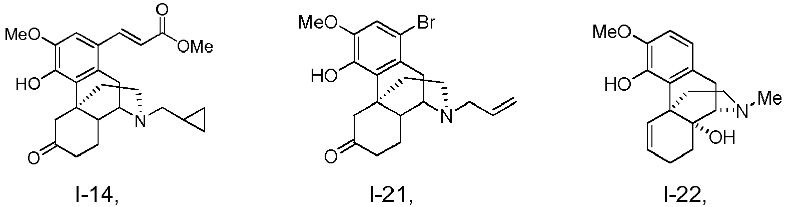 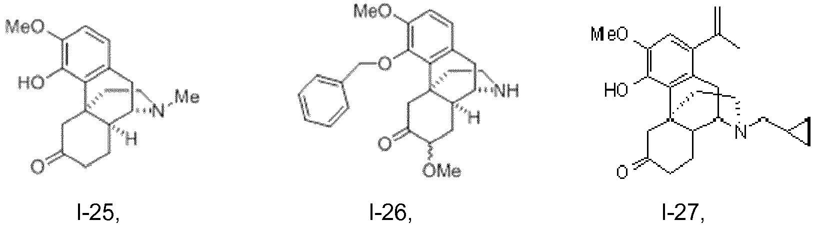 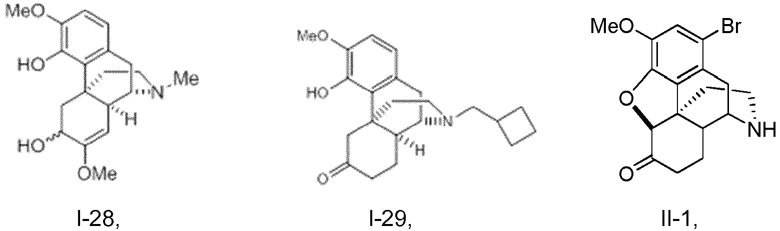 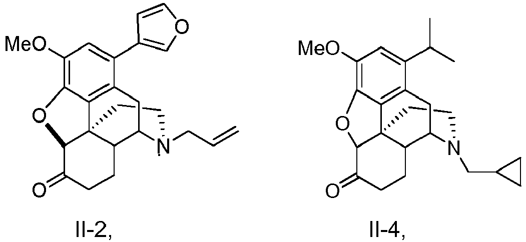 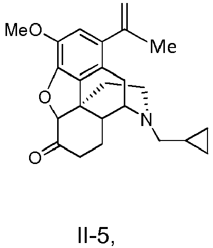 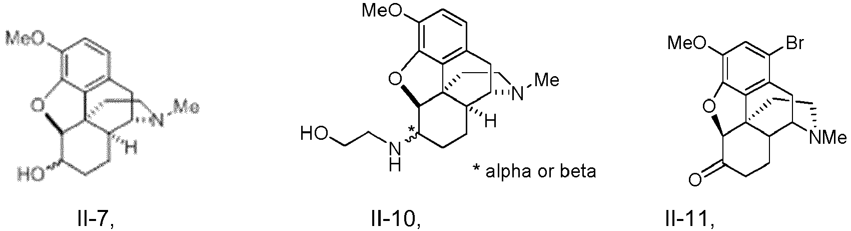 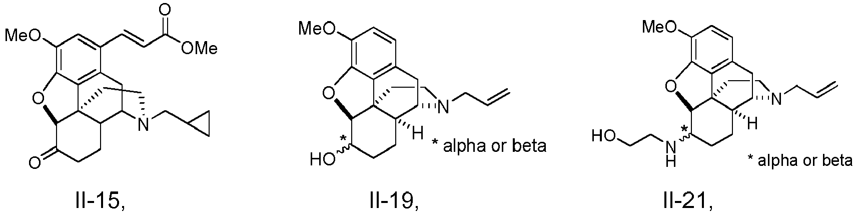 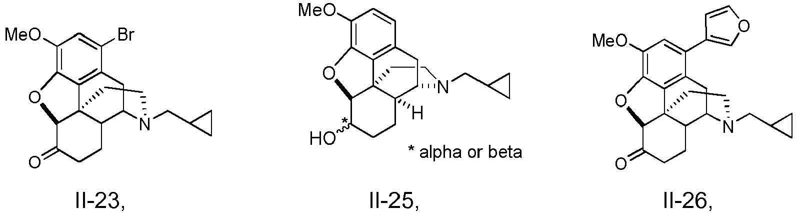 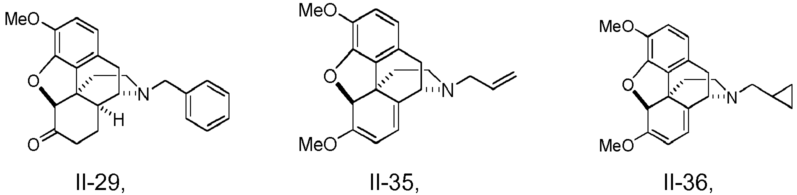 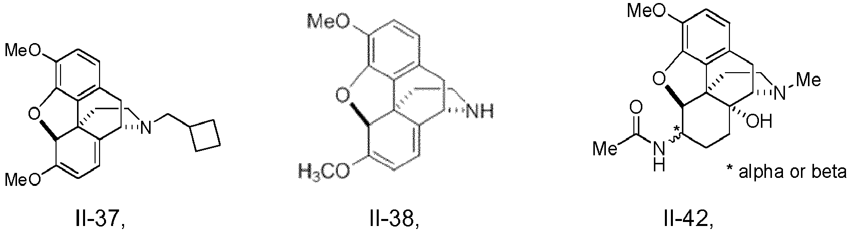 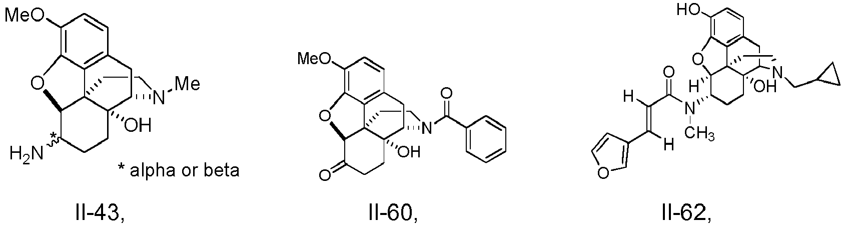 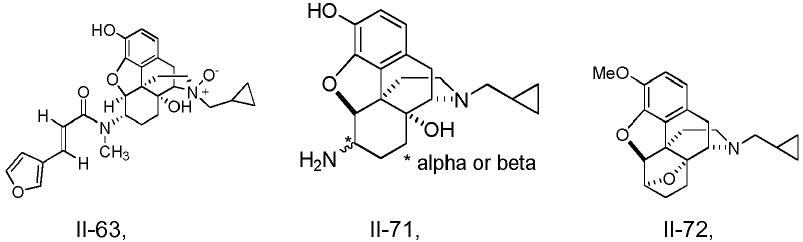 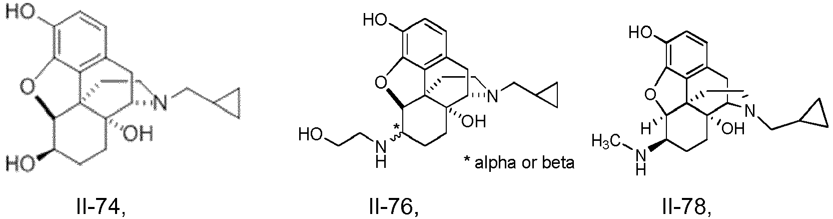 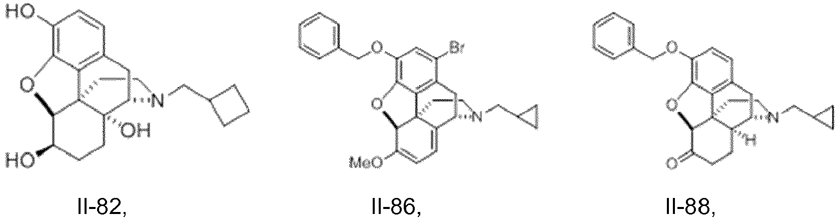 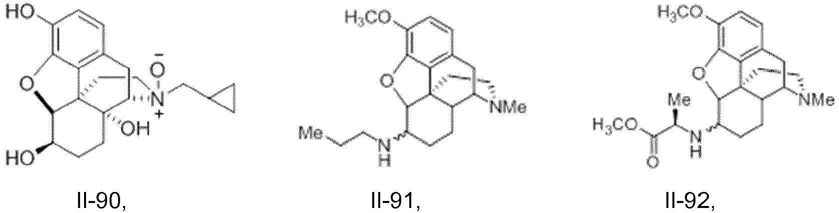 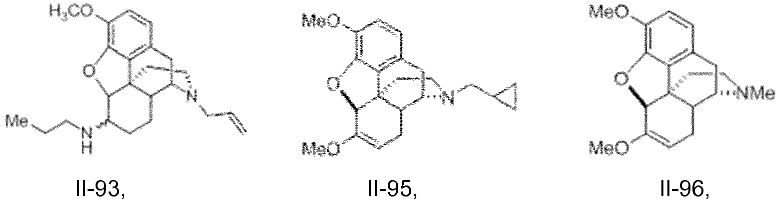 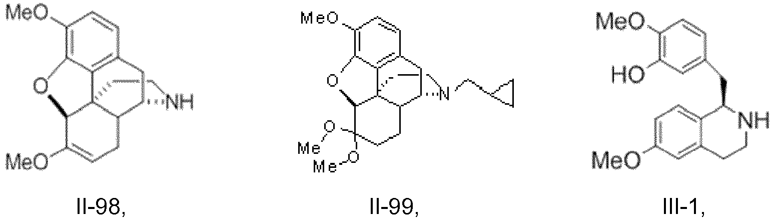 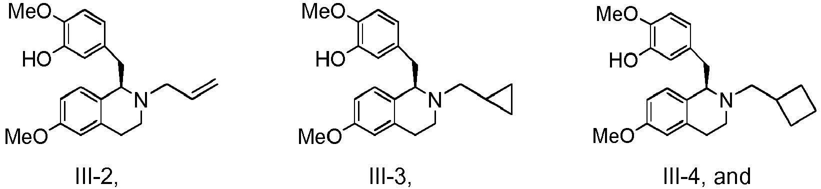 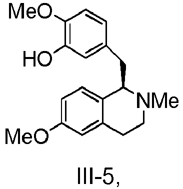 or a pharmaceutically acceptable salt thereof.

3. The method of claim 2, wherein the cell is chosen from a glial cell, a microglial cell, and an astrocyte.

## Patentansprüche

1. Verbindung, die ausgewählt ist aus den folgenden Formeln: 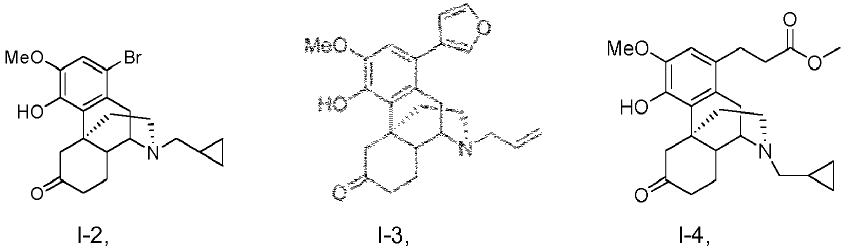 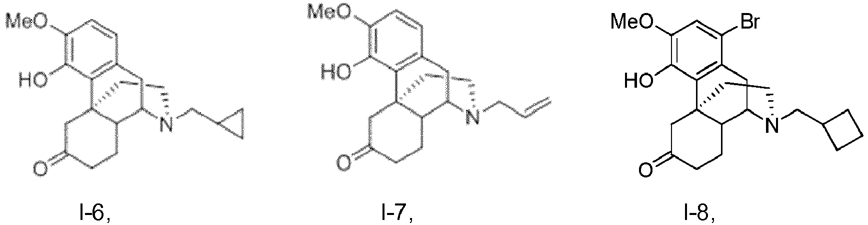 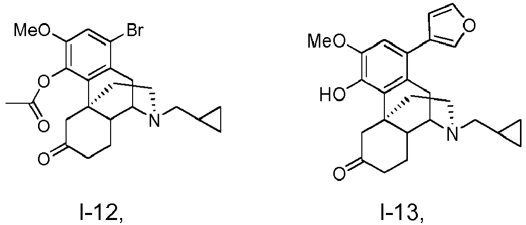 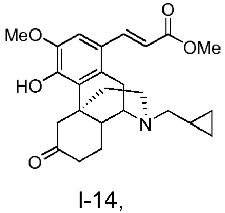 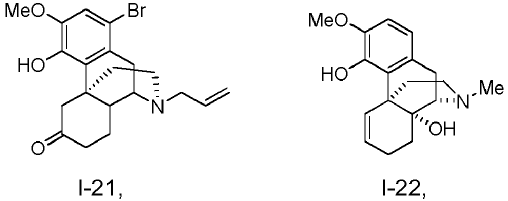 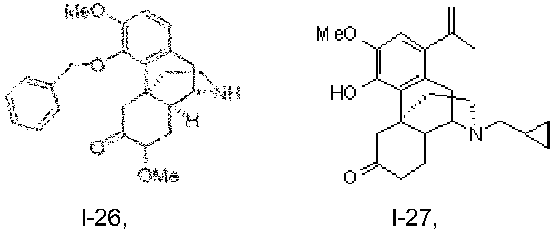 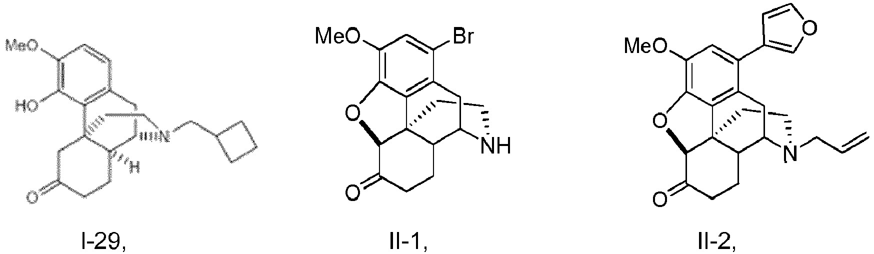 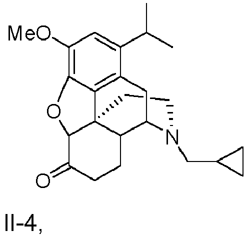 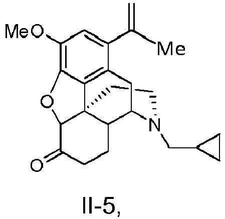 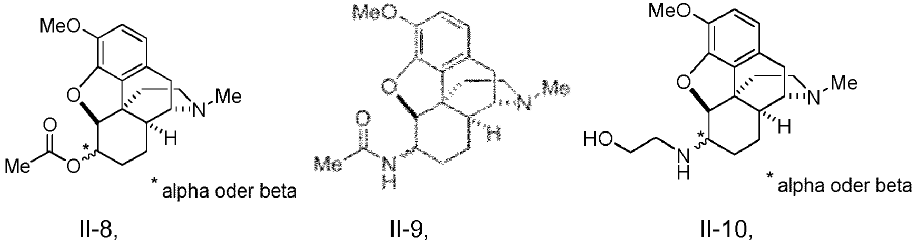 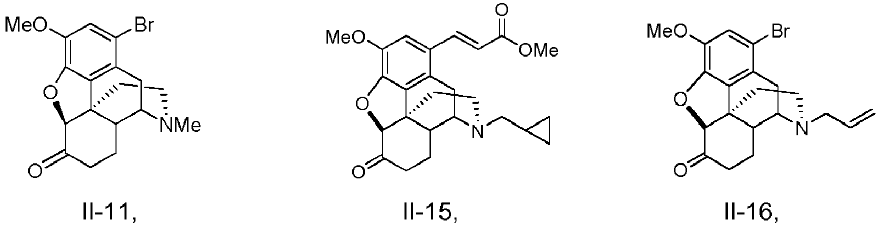 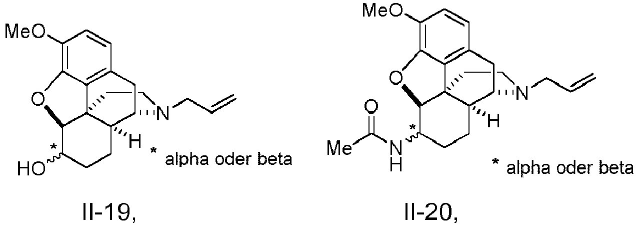 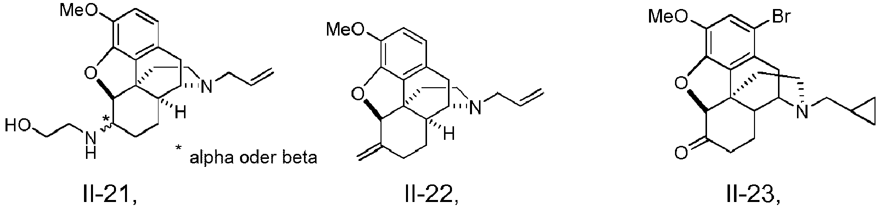 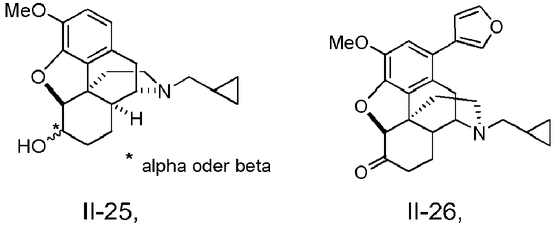 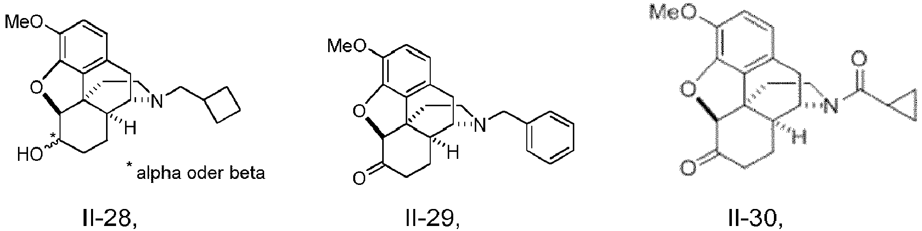 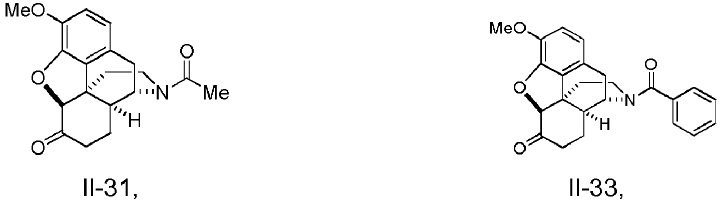 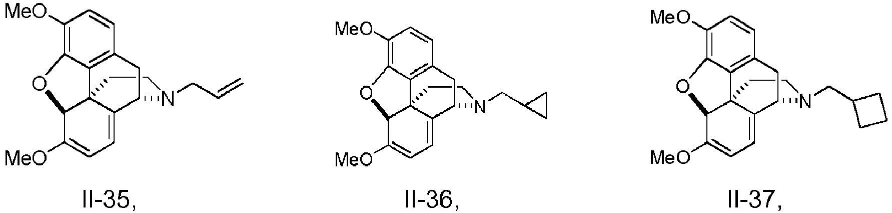 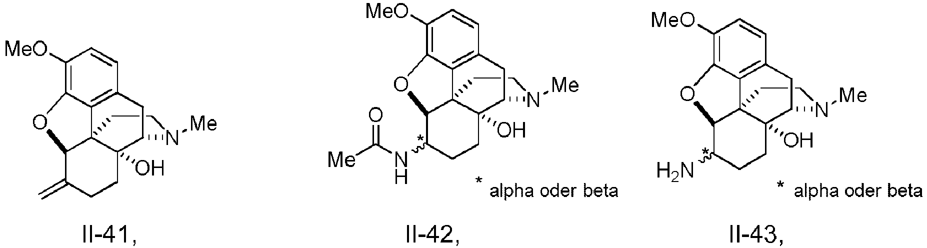 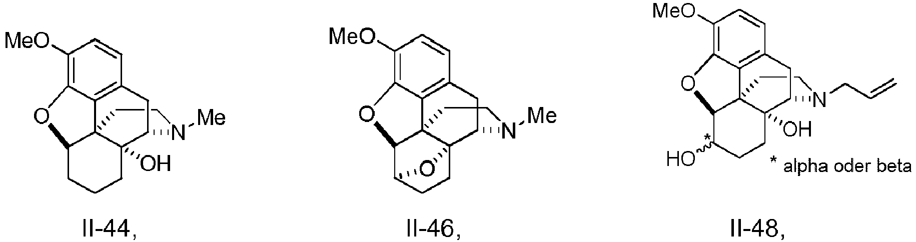 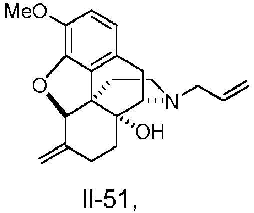 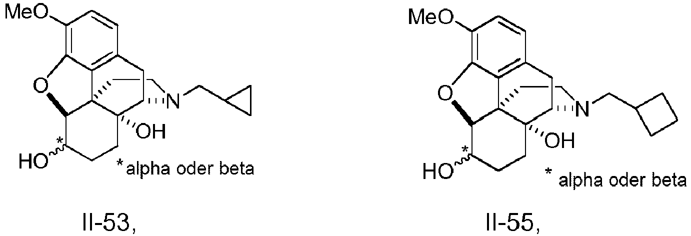 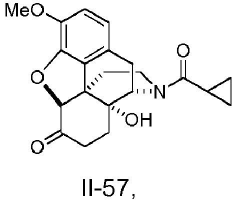 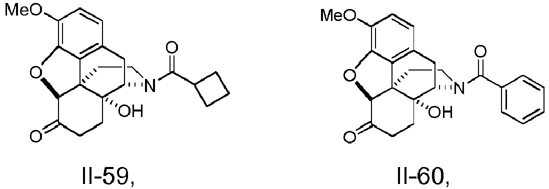 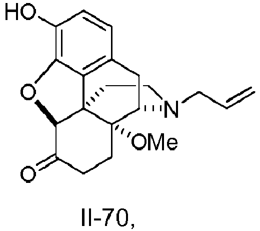 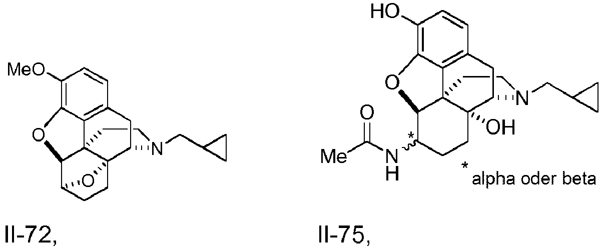 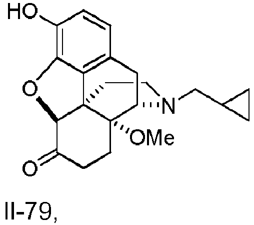 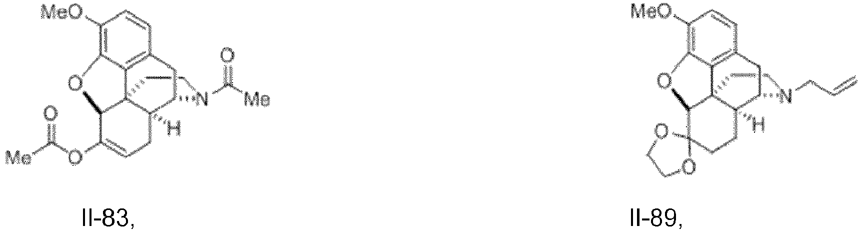 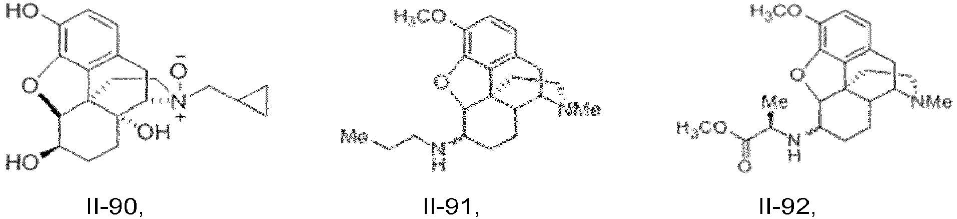 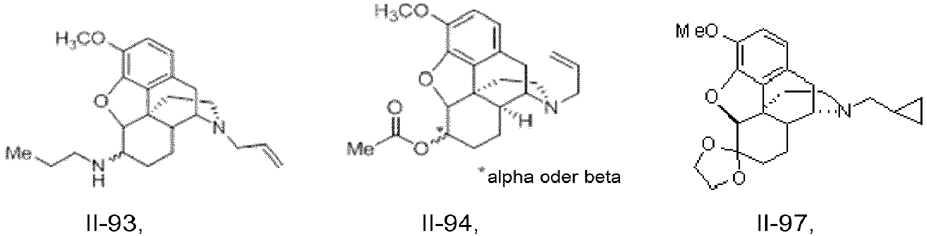 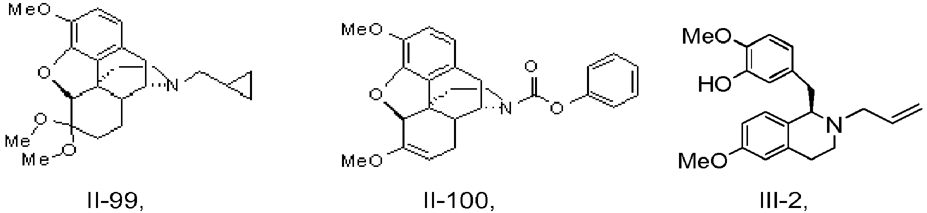 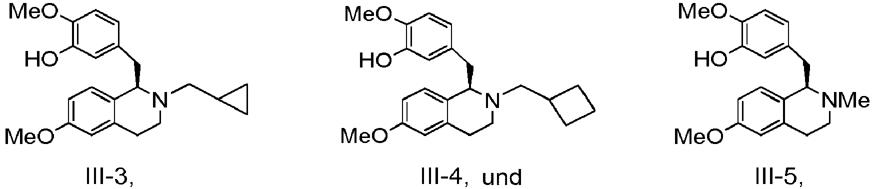 oder ein pharmazeutisch verträgliches Salz davon.

2. Verfahren zum Hemmen von TLR9-Aktivierung, wobei das Verfahren In-Kontaktbringen einer TLR9 exprimierenden Zelle mit einer Verbindung umfasst, die ausgewählt ist aus den folgenden Formeln: 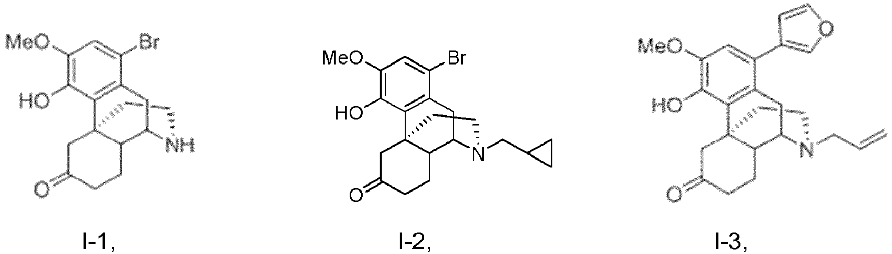 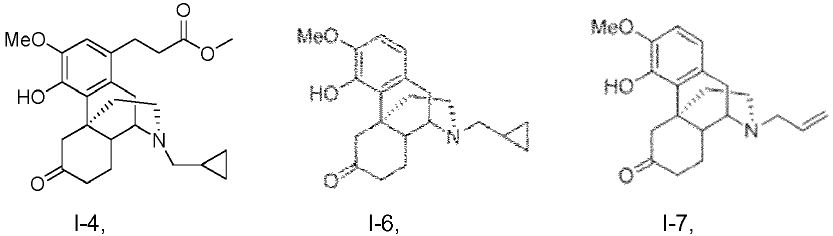 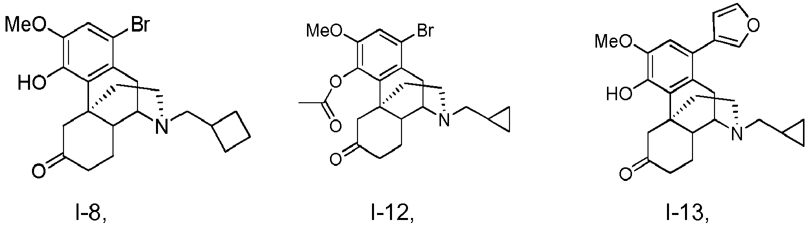 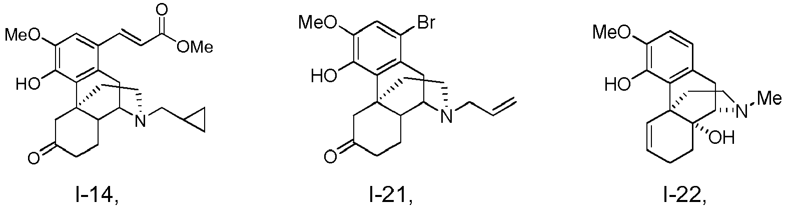 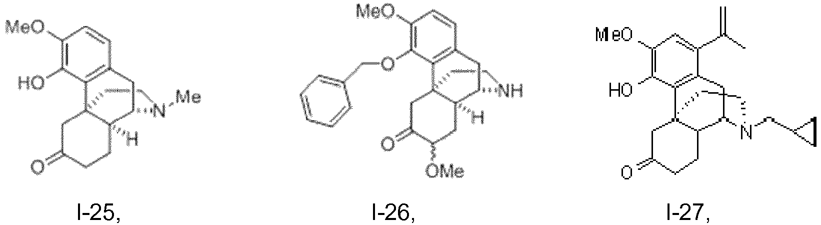 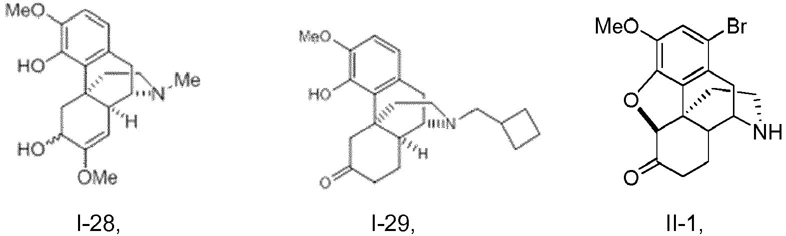 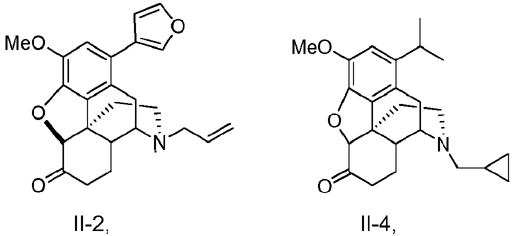 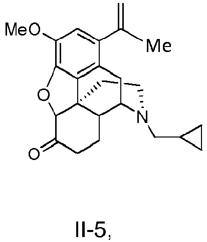 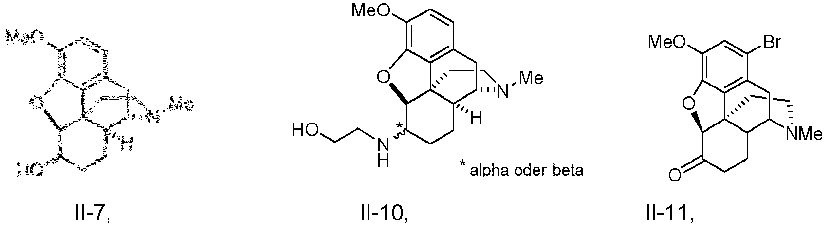 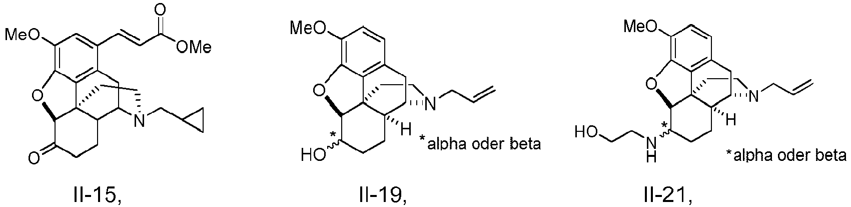 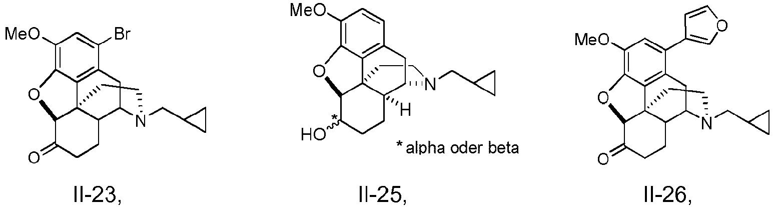 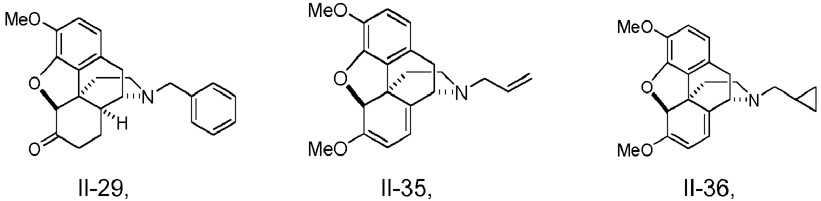 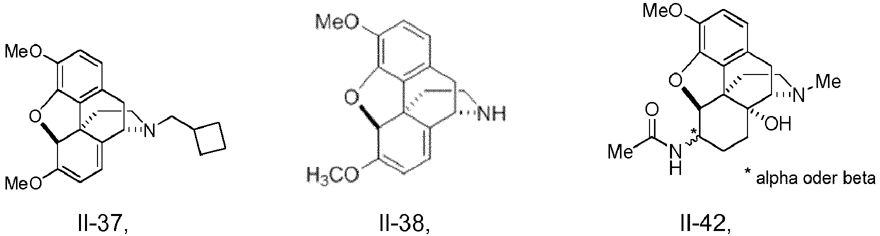 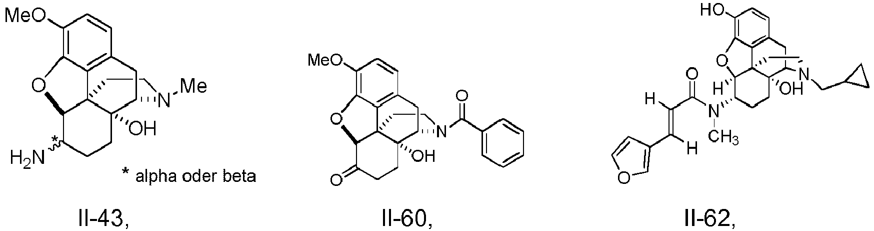 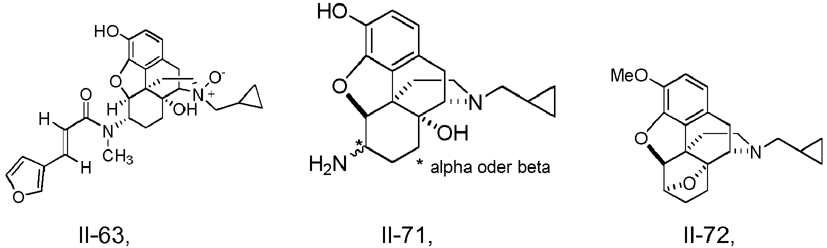 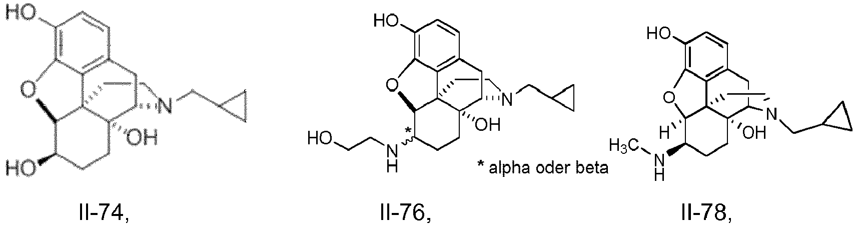 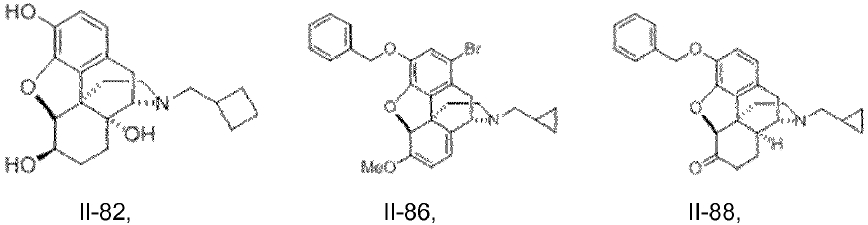 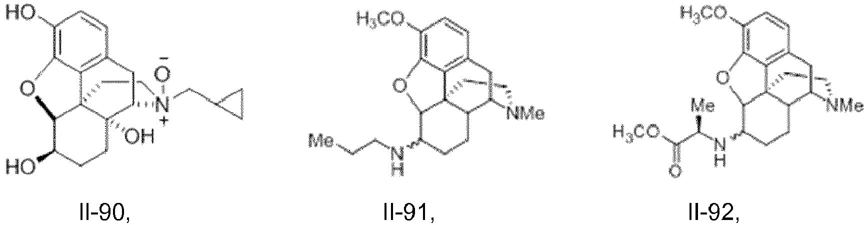 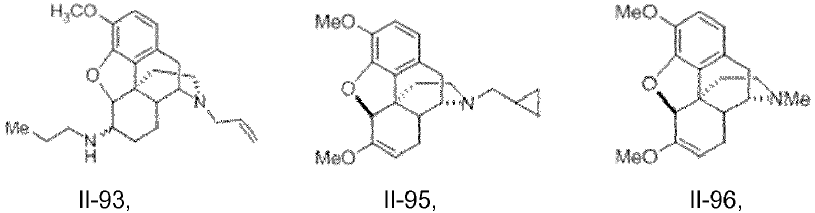 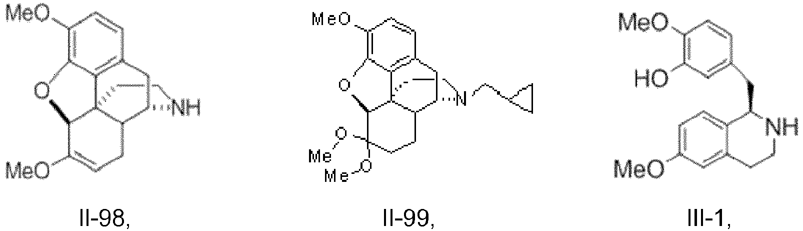 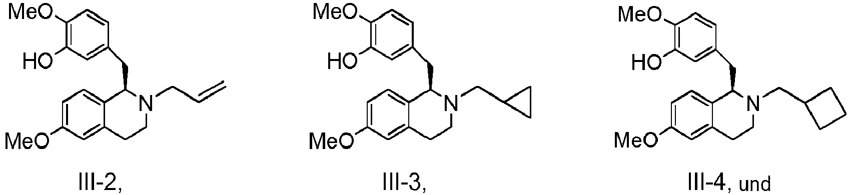 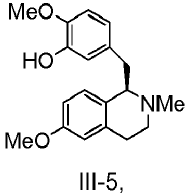 oder einem pharmazeutisch verträglichen Salz davon.

3. Verfahren nach Anspruch 2, wobei die Zelle ausgewählt ist aus einer Gliazelle, einer Mikrogliazelle und einem Astrozyten.

## Revendications

1. Composé choisi parmi les formules suivantes: 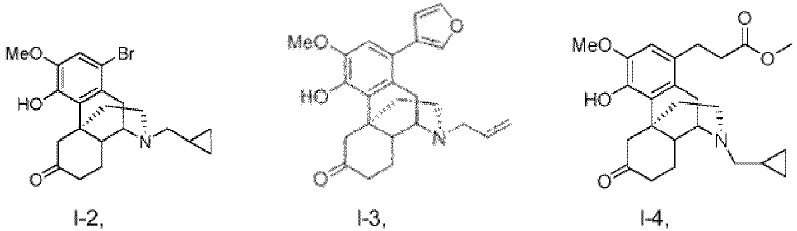 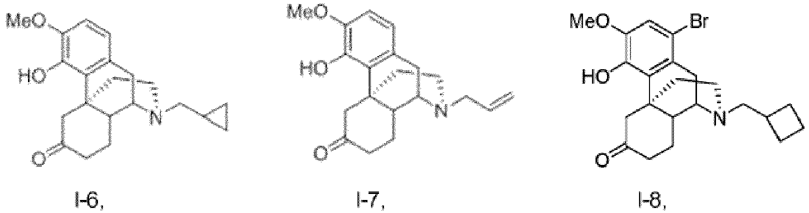 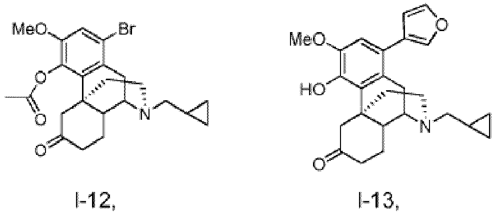 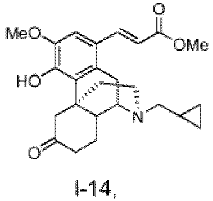 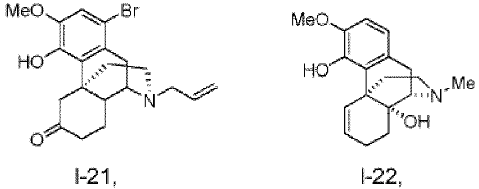 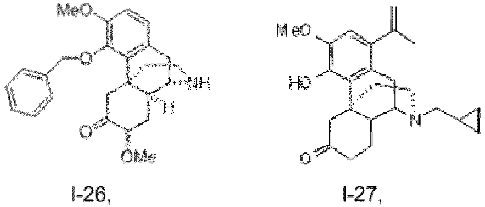 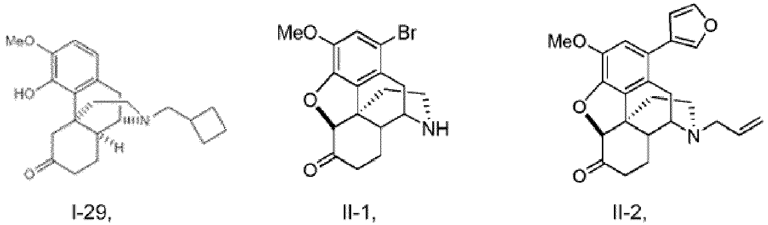 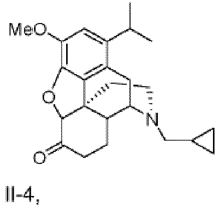 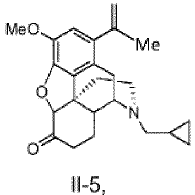 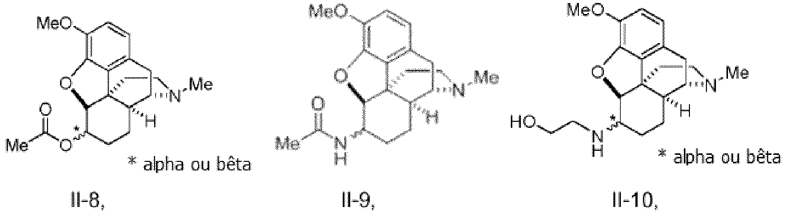 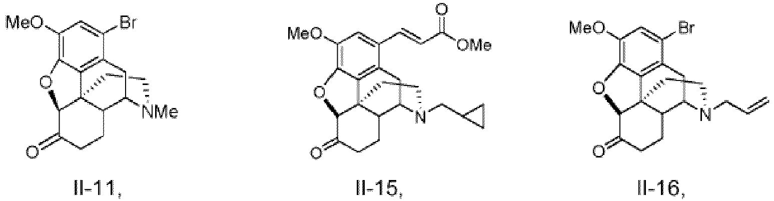 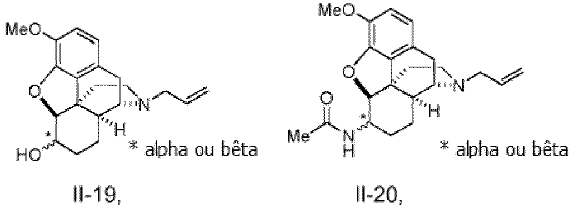 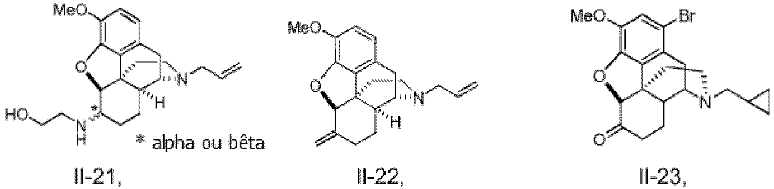 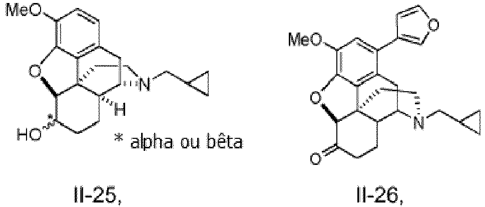 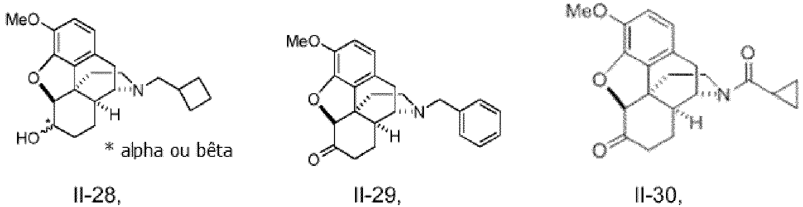 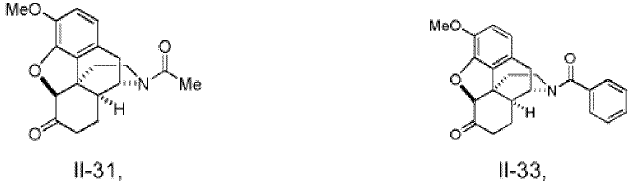 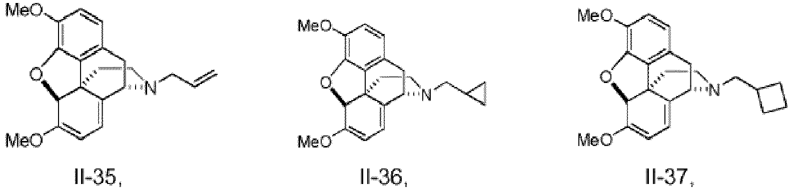 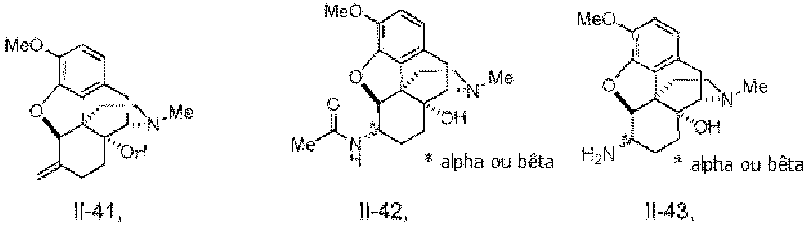 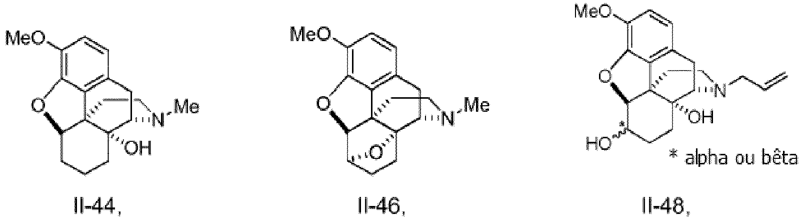 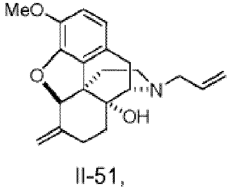 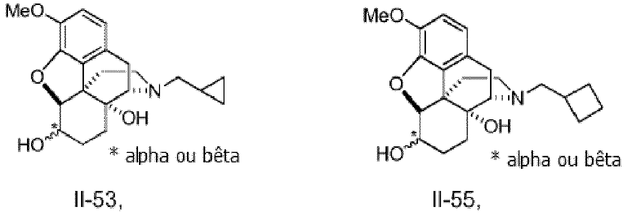 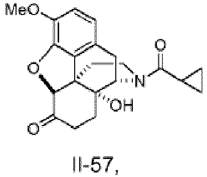 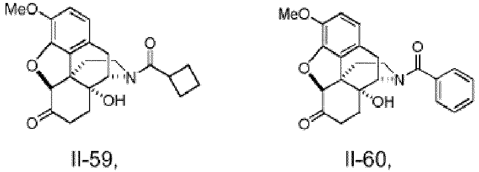 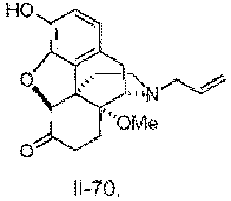 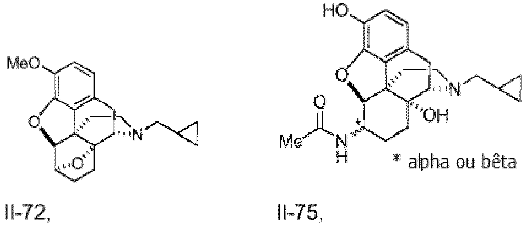 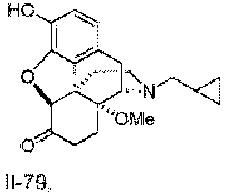 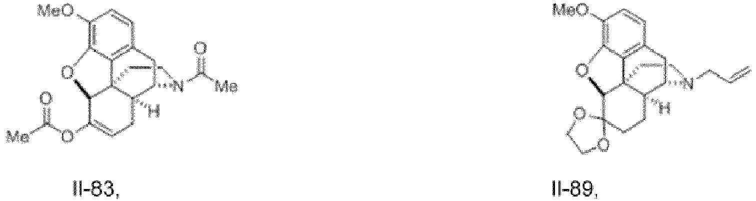 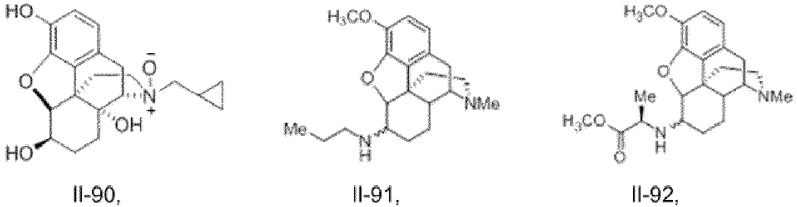 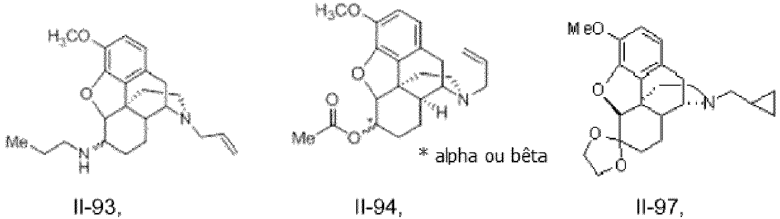 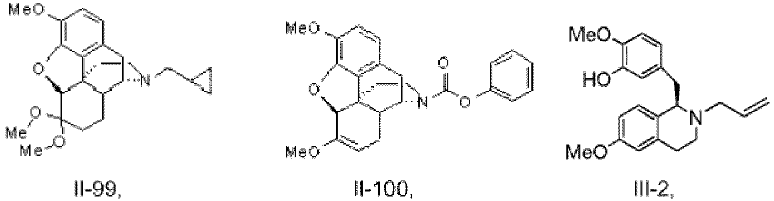 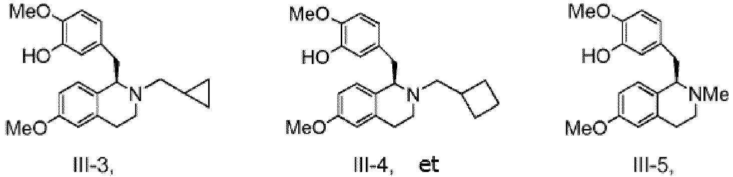 ou sel pharmaceutiquement acceptable de celui-ci.

2. Procédé pour inhiber l'activation de TLR9, le procédé comprenant la mise en contact *in vitro* d'une cellule exprimant TLR9 avec un composé choisi parmi les formules suivantes:

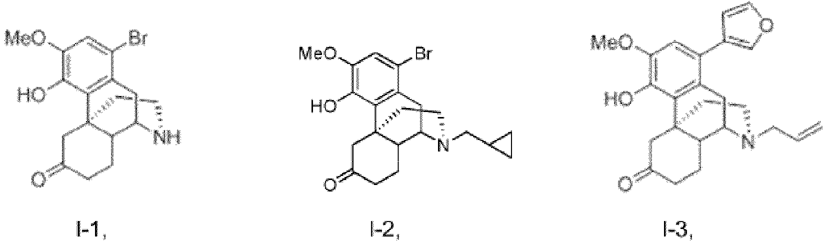

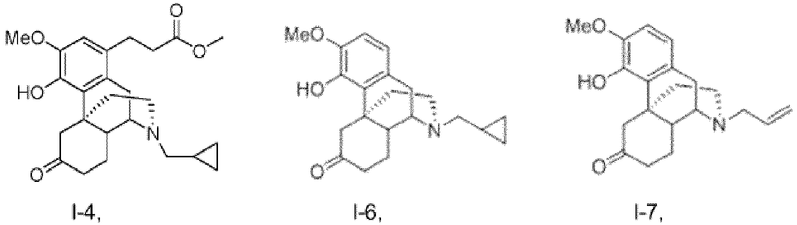

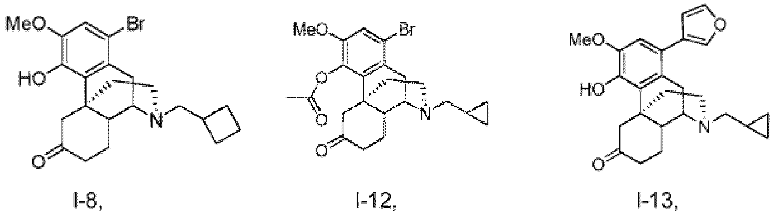

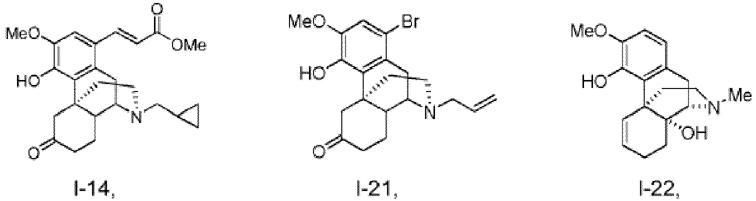

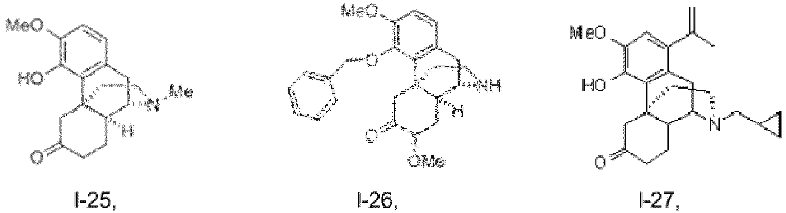

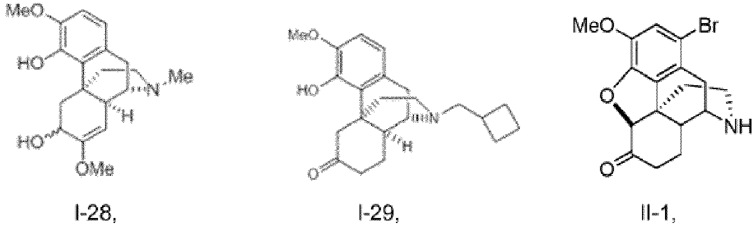

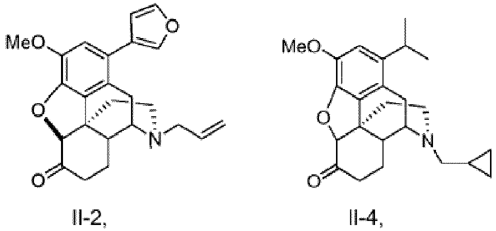

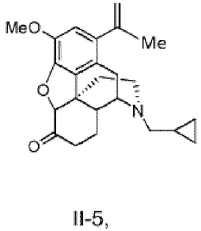

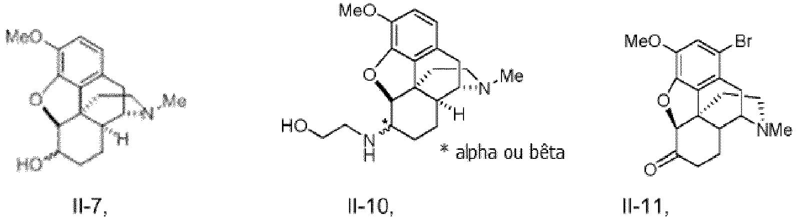

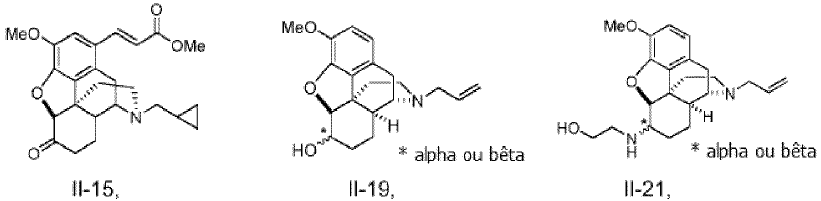

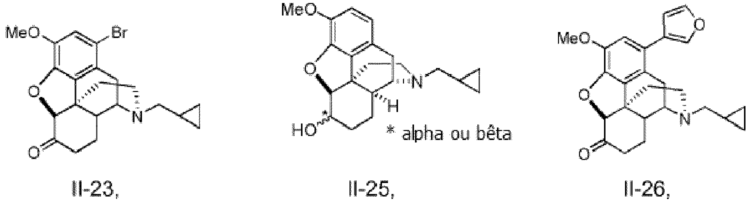

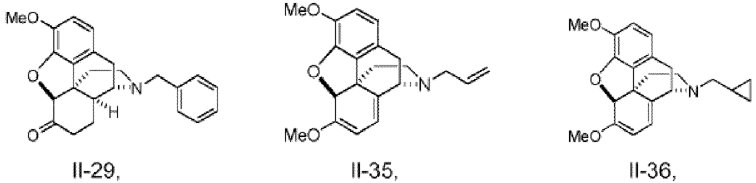

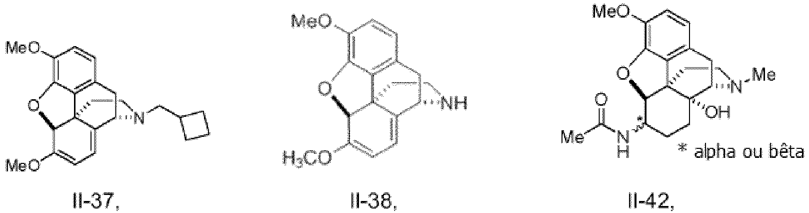

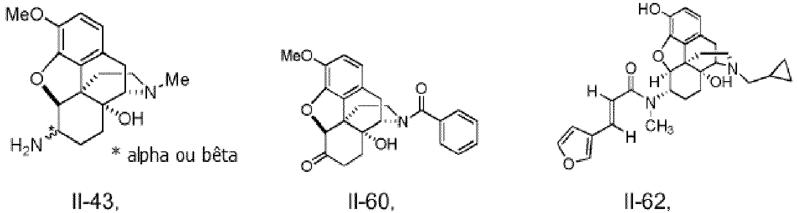

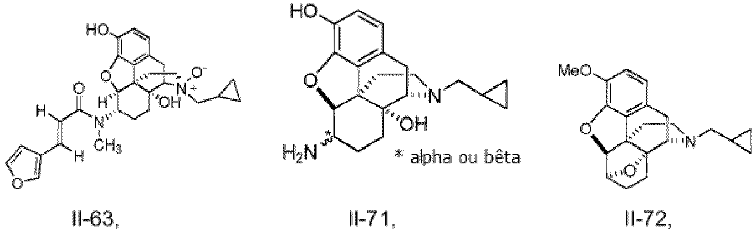

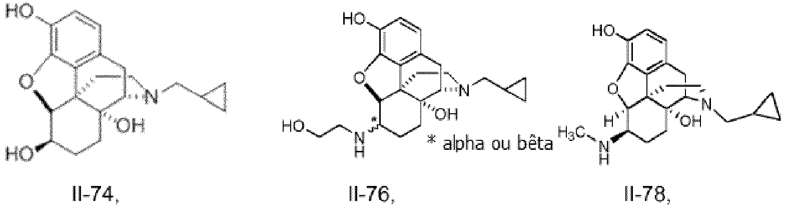

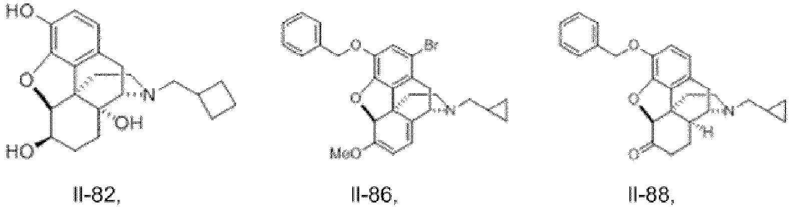

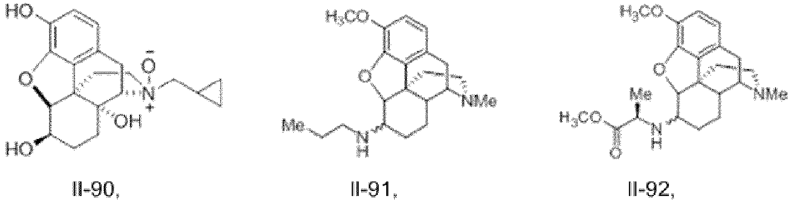

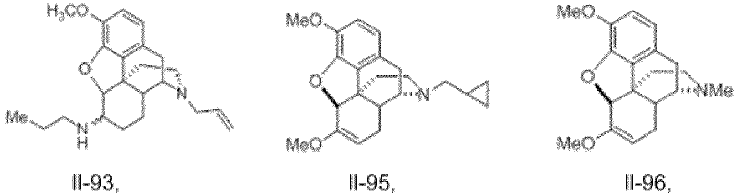

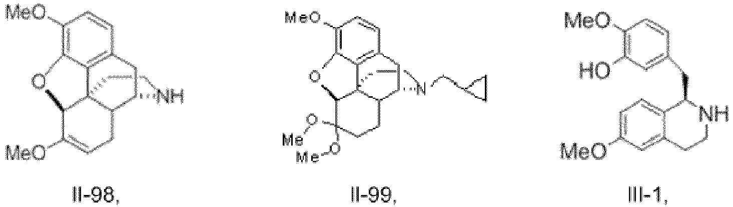

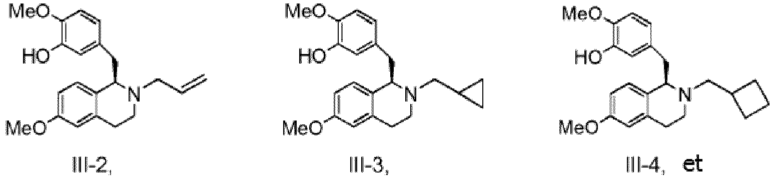

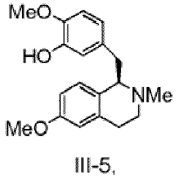

3. Procédé selon la revendication 2, où la cellule est choisie parmi une cellule gliale, une cellule microgliale et un astrocyte.
